(19) Europäisches Patentamt
European Patent Office
Office européen des brevets

(11) **EP 4 610 986 A1**

(12) **EUROPEAN PATENT APPLICATION**
published in accordance with Art. 153(4) EPC

(43) Date of publication:
**03.09.2025 Bulletin 2025/36**

(21) Application number: **23897887.8**

(22) Date of filing: **30.11.2023**

(51) International Patent Classification (IPC):
**G16B 25/00** (2019.01)     **C12M 1/00** (2006.01)
**C12M 1/34** (2006.01)     **C12N 15/09** (2006.01)

(52) Cooperative Patent Classification (CPC):
**C12M 1/00; C12M 1/34; C12N 15/09; G16B 25/00**

(86) International application number:
**PCT/JP2023/043019**

(87) International publication number:
**WO 2024/117241 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.12.2022 JP 2022193737**

(71) Applicant: **FUJIFILM Corporation
Tokyo 106-8620 (JP)**

(72) Inventor: **SUZUKI, Takafumi
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54) **INFORMATION PROCESSING APPARATUS, OPERATION METHOD FOR INFORMATION PROCESSING APPARATUS, AND OPERATION PROGRAM FOR INFORMATION PROCESSING APPARATUS**

(57)     An information processing device executes processing of detecting a differential expressed gene that exhibits a specific expression with respect to a cell characteristic of interest, based on gene expression level data of a cell population in which a plurality of subtypes are mixed, and the information processing device includes a processor in which the processor assigns a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene, and searches for a first probability distribution that fits the distributions of the gene expression levels of the two groups for each of the plurality of candidate genes, based on an assignment result of the clusters.

FIG. 5

**Description**

BACKGROUND OF THE INVENTION

1. Field of the Invention

The technology of the present disclosure relates to an information processing device, an operation method of an information processing device, and an operation program of an information processing device.

2. Description of the Related Art

As a technology that is the core of gene analysis, a technology for detecting a differential expressed gene (hereinafter, referred to as a DEG) that exhibits a specific expression with respect to a cell characteristic of interest based on gene expression level data of a certain cell population is known.

As an example, a case is considered in which a gene (referred to as an ER marker gene) that is important for determining a state of an estrogen receptor (hereinafter, referred to as an ER) as the cell characteristic of interest is detected based on gene expression level data of cancer tissue-derived cells sampled from a breast invasive cancer (hereinafter, referred to as BRCA) patient. In such a case, the BRCA patients are divided into two groups depending on the magnitude of the proportion of the cancer tissue-derived cells having the ER, and the gene expression levels of the cancer tissue-derived cells are compared between the groups to detect the ER marker gene.

In addition, as another example, a case is considered in which a gene having a high contribution to antibody production as the cell characteristic of interest is detected based on gene expression level data of Chinese hamster ovary (hereinafter, referred to as CHO) cells widely used for producing an antibody pharmaceutical. In such a case, a population of the CHO cells is divided into two groups depending on an amount of the antibody production, and the gene expression levels are compared between the groups to detect the gene having a high contribution to the antibody production.

A method of detecting the DEG, called edgeR, is described in <M. D. Robinson, et al., "edgeR: a Bioconductor package for differential expression analysis of digital gene expression data." Bioinformatics 26, 139 2010.> (hereinafter, referred to as Non-Patent Literature 1). The method of detecting the DEG described in Non-Patent Literature 1 is an algorithm constructed based on a finding that the distribution of the gene expression levels of the cell population (a histogram in which a horizontal axis is the gene expression level and a vertical axis is the number of the samples) well follows the negative binomial distribution.

In the method of detecting the DEG described in Non-Patent Literature 1, a negative binomial distribution that fits the distribution of the gene expression levels of two groups of the cell population is searched for. Next, based on parameters such as the mean and the variance of the two negative binomial distributions that are searched for, it is determined whether or not there is a large difference between the groups by a statistical method, and a gene determined to have a large difference between the groups is detected as the DEG. It should be noted that processing of searching for a probability distribution that fits a certain distribution, such as searching for the negative binomial distribution that fits the distribution of the gene expression levels, is called statistical modeling.

SUMMARY OF THE INVENTION

Some cell populations have a plurality of subtypes mixed therein. In such a cell population in which the plurality of subtypes are mixed, it is expected that a plurality of clusters (peaks) due to the plurality of subtypes appear in the distribution of the gene expression levels. On the other hand, the method of detecting the DEG described in Non-Patent Literature 1 assumes a so-called unimodal distribution of gene expression levels having one cluster, and does not assume a so-called multimodal distribution of gene expression levels having a plurality of clusters. Therefore, in the method of detecting the DEG described in Non-Patent Literature 1, there is a concern that an appropriate DEG cannot be detected.

One embodiment according to the technology of the present disclosure provides an information processing device, an operation method of an information processing device, and an operation program of an information processing device capable of detecting an appropriate DEG even in a case in which a cell population in which a plurality of subtypes are mixed is an analysis target.

The present disclosure relates to an information processing device that executes processing of detecting a differential expressed gene that exhibits a specific expression with respect to a cell characteristic of interest, based on gene expression level data of a cell population in which a plurality of subtypes are mixed, the information processing device comprising: a processor, in which the processor assigns a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene, and searches for a first probability distribution that fits the distributions of the gene expression levels of the two groups for each of the plurality of candidate genes, based on an assignment result of the clusters.

It is preferable that the processor performs a determination of whether or not there is a statistically significant difference in the distribution of the gene expression levels between the two groups, based on a search result of the first probability distribution, for each of the plurality of candidate genes, and detects the candidate gene for which it is determined that there is the statistically significant difference in the distribution of the gene expression levels between the two groups, as the differential expressed gene.

It is preferable that the processor calculates a representative index value for each of the two groups from the distribution of the gene expression levels and the searched first probability distribution, calculates a score representing a degree of a difference between the two groups from the representative index value, and performs the determination based on the score.

It is preferable that the processor performs, at least once, processing of re-assigning the clusters by receiving a search result of the first probability distribution and re-searching for the first probability distribution based on a re-assignment result of the clusters.

It is preferable that the processor changes the number of the clusters to be assigned, to search for the first probability distribution for each changed number, and determines a number in which the first probability distribution that best fits the distribution of the gene expression levels is searchable for from among the changed numbers, based on a search result of the first probability distribution.

It is preferable that the processor assigns the clusters by using a second probability distribution.

It is preferable that the first probability distribution is a negative binomial distribution.

It is preferable that the processor generates simulated gene expression level data by simulation, based on the searched first probability distribution.

It is preferable that the processor generates only the simulated gene expression level data of the differential expressed gene by the simulation.

An operation method of an information processing device that executes processing of detecting a differential expressed gene that exhibits a specific expression with respect to a cell characteristic of interest, based on gene expression level data of a cell population in which a plurality of subtypes are mixed, includes: assigning a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene; and searching for a first probability distribution that fits the distributions of the gene expression levels of the two groups for each of the plurality of candidate genes, based on an assignment result of the clusters.

An operation program of an information processing device that executes processing of detecting a differential expressed gene that exhibits a specific expression with respect to a cell characteristic of interest, based on gene expression level data of a cell population in which a plurality of subtypes are mixed, causes a computer to execute a process comprising: assigning a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene; and searching for a first probability distribution that fits the distributions of the gene expression levels of the two groups for each of the plurality of candidate genes, based on an assignment result of the clusters.

According to the technology of the present disclosure, it is possible to provide the information processing device, the operation method of the information processing device, and the operation program of the information processing device capable of detecting an appropriate DEG even in a case in which the cell population in which the plurality of subtypes are mixed is the analysis target.

BRIEF DESCRIPTION OF THE DRAWINGS

Fig. 1 is a diagram showing ER state information of a BRCA patient, gene expression level data of cancer tissue-derived cells sampled from the BRCA patient, and an information processing device.
Fig. 2 is a diagram showing a state in which the BRCA patients are divided into a high-expression group and a low-expression group based on the ER state information, and a gene expression level distribution.
Fig. 3 is a block diagram of a computer constituting the information processing device.
Fig. 4 is a block diagram showing a processing unit of a CPU of the information processing device.
Fig. 5 is a diagram showing processing of an assignment unit.
Fig. 6 is a diagram showing a negative binomial distribution that fits a gene expression level distribution.
Fig. 7 is a diagram showing a search result.
Fig. 8 is a flowchart showing a procedure of processing of a detection unit.

Fig. 9 is a diagram showing a detailed configuration of the detection unit.

Fig. 10 is a diagram showing a function calculation result.

Fig. 11 is a diagram showing a score calculation result.

Fig. 12 is a flowchart showing a procedure of processing of a determination unit.

Fig. 13 is a diagram showing a gene analysis screen on which a detection result of a DEG is displayed.

Fig. 14 is a diagram showing a state in which the gene expression level of the DEG among the gene expression levels of all genes is input to an ER state prediction model and an ER state prediction result is output from the ER state prediction model.

Fig. 15 is a flowchart showing a procedure of processing of the information processing device.

Fig. 16 is a diagram showing a second embodiment in which clusters are assigned by using a categorical distribution.

Fig. 17 is a diagram showing a third embodiment in which a search result of a negative binomial distribution obtained by a search unit is fed back to an assignment unit and causes the assignment unit to re-assign the clusters.

Fig. 18 is a flowchart showing a procedure of processing of the information processing device in the third embodiment.

Fig. 19 is a diagram showing a fourth embodiment in which the number of the clusters to be assigned is changed and the negative binomial distribution is searched for, for each changed number.

Fig. 20 is a diagram showing a state in which a number in which the negative binomial distribution that best fits the gene expression level distribution is searchable for is determined from among the changed numbers.

Fig. 21 is a diagram showing a 5_1st embodiment in which simulated gene expression level data is generated by simulation based on the searched negative binomial distribution.

Fig. 22 is a diagram showing a 5_2nd embodiment in which only the simulated gene expression level data of the DEG is generated by the simulation.

Fig. 23 is a table showing a breakdown of groups and subtypes of samples in Example.

Fig. 24 is a diagram showing a gene expression level distribution of a gene detected as the DEG in both the related-art technology and the technology of the present disclosure.

Fig. 25 is a diagram showing a gene expression level distribution of a gene that is detected as the DEG in the related-art technology but is not detected as the DEG in the technology of the present disclosure.

Fig. 26 is a diagram showing a gene expression level distribution of a gene that is detected as the DEG in the technology of the present disclosure but is not detected as the DEG in the related-art technology.

## DESCRIPTION OF THE PREFERRED EMBODIMENTS

[First Embodiment]

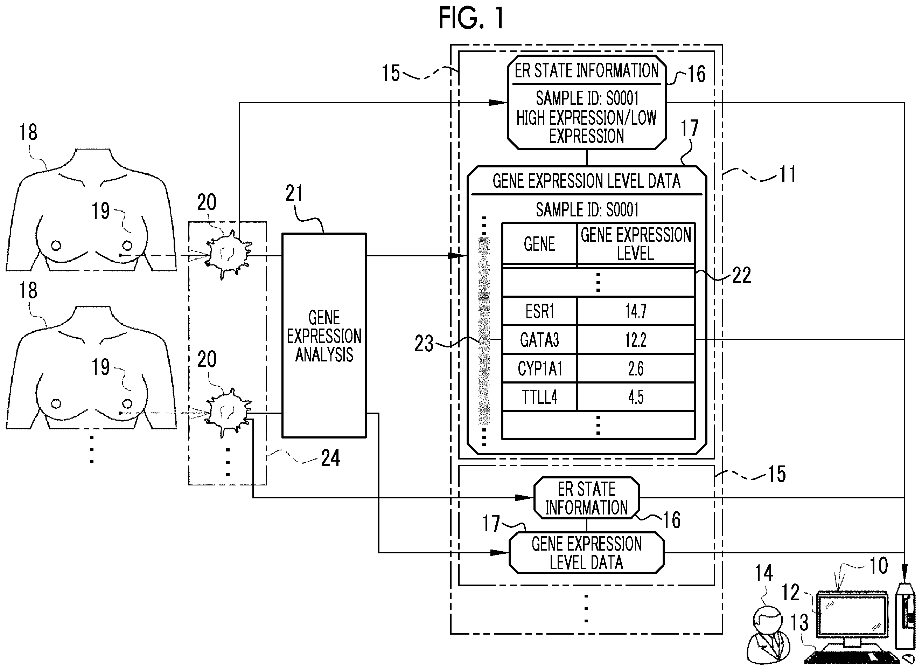

As shown in Fig. 1 as an example, the information processing device 10 according to the technology of the present disclosure executes processing of detecting a DEG (ER marker gene) based on a sample data group 11. The information processing device 10 is, for example, a desktop personal computer, and comprises a display 12 that displays various screens and an input device 13, such as a keyboard, a mouse, a touch panel, and/or a microphone for audio input. The information processing device 10 is installed in, for example, a gene analysis facility and is operated by a user 14 who is involved in a gene analysis in the gene analysis facility.

The sample data group 11 is input to the information processing device 10. The sample data group 11 includes a plurality of pieces of sample data 15. The sample data 15 is composed of a set of ER state information 16 and gene expression level data 17. One sample data 15, that is, one set of the ER state information 16 and the gene expression level data 17 is derived from one BRCA patient 18.

The ER state information 16 is information indicating a state of expression of an ER of cancer tissue-derived cells 20 sampled from a breast 19 of the BRCA patient 18. In a case in which a proportion of the cancer tissue-derived cells 20 having the ER is greater than a preset threshold value, "high expression" is registered in the ER state information 16. On the other hand, in a case in which the proportion of the cancer tissue-derived cells 20 having the ER is less than the threshold value, "low expression" is registered in the ER state information 16. The ER state information 16 includes identification data (ID) for uniquely identifying individual BRCA patients 18. The state of expression of the ER in the cancer tissue-derived cells 20 registered in the ER state information 16 is an example of a "cell characteristic of interest" according to the technology of the present disclosure.

The gene expression level data 17 is obtained by subjecting the cancer tissue-derived cells 20 to a gene expression analysis 21, such as ribonucleic acid (RNA)-sequencing (Seq). As shown in Table 22, the gene expression level is registered in the gene expression level data 17 for each gene. A heat map 23 visualizes the gene expression level for each gene by representing the gene expression level with the type and shade of color.

Similar to the ER state information 16, the gene expression level data 17 also includes the sample ID. That is, the ER state information 16 and the gene expression level data 17 are associated with each other by the sample ID.

A plurality of cancer tissue-derived cells 20 sampled from a plurality of BRCA patients 18 constitute a cancer tissue-derived cell population 24. The cancer tissue-derived cells 20 have four subtypes of "Basal-like", "Human Epidermal Growth Factor Receptor Type 2 (HER2)-enriched", "Luminal A", and "Luminal B". In addition, the presence of the subtype referred to as "Normal-like" has also been suggested for the cancer tissue-derived cells 20 in a plurality of literatures. That is, the cancer tissue-derived cell population 24 is an example of a "cell population in which a plurality of subtypes are mixed" according to the technology of the present disclosure. It should be noted that, in a case in which the number of the plurality of cancer tissue-derived cells 20 (the number of the samples) constituting the cancer tissue-derived cell population 24 is denoted by N, and the number of the genes is denoted by G, the gene expression level data 17 can be represented by a matrix $Y \in NN^{G \times N}$ in which each element has a value of a natural number NN.

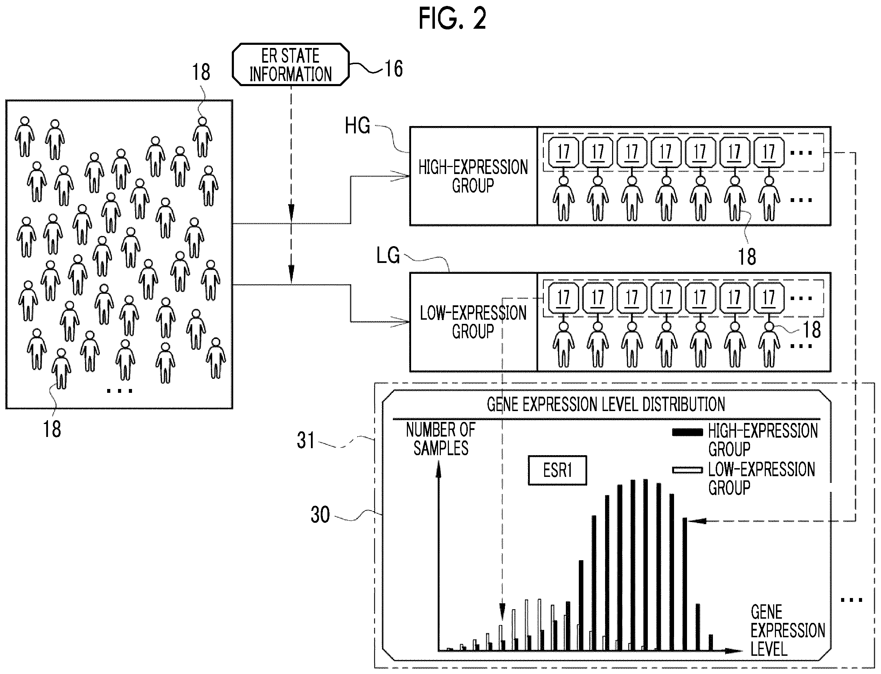

As shown in Fig. 2 as an example, the BRCA patients 18 are divided into any of two groups of a high-expression group HG and a low-expression group LG, based on the ER state information 16. Specifically, the BRCA patient 18 in which "high expression" is registered in the ER state information 16 is divided into the high-expression group HG. On the other hand, the BRCA patient 18 in which "low expression" is registered in the ER state information 16 is divided into the low-expression group LG. For each of the high-expression group HG and the low-expression group LG, it is possible to generate a distribution of gene expression levels (hereinafter, referred to as a gene expression level distribution) 30 for each of a plurality of candidate genes of the DEG based on the gene expression level data 17. The gene expression level distribution 30 is a histogram in which a horizontal axis is a gene expression level of the candidate gene and a vertical axis is the number of the samples i. In Fig. 2, the gene expression level distribution 30 of the candidate gene ESR1 is shown. Hereinafter, a collection of the gene expression level distributions 30 generated for each of the plurality of candidate genes will be referred to as a gene expression level distribution group 31. It should be noted that the candidate gene is a gene selected in advance by the user 14 from all genes. The number of the candidate genes is, for example, about 20000.

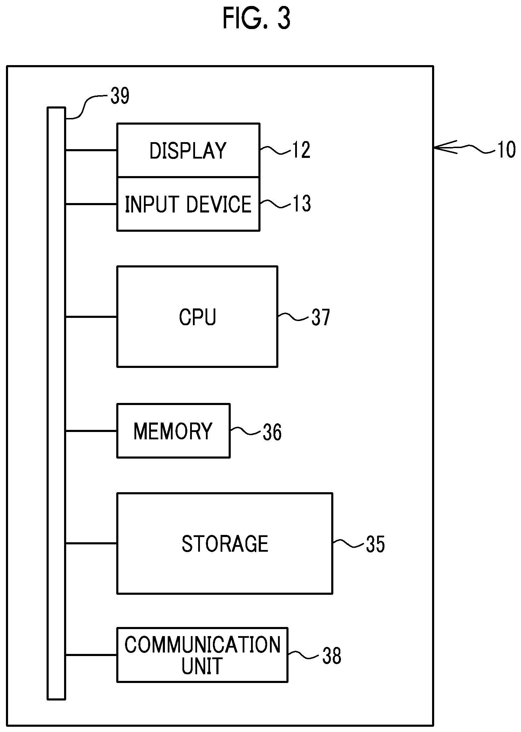

For example, as shown in Fig. 3, a computer constituting the information processing device 10 comprises a storage 35, a memory 36, a central processing unit (CPU) 37, and a communication unit 38, in addition to the display 12 and the input device 13 described above. These units are connected to each other via a busline 39.

The storage 35 is a hard disk drive that is built in the computer constituting the information processing device 10 or connected to the computer through a cable or a network. Alternatively, the storage 35 is a disk array in which a plurality of hard disk drives are mounted. The storage 35 stores a control program such as an operating system, various application programs, various types of data associated with these programs, and the like. It should be noted that a solid state drive may be used instead of the hard disk drive.

The memory 36 is a work memory for the CPU 37 to execute processing. The CPU 37 loads the program stored in the storage 35 into the memory 36, and executes processing in accordance with the program. As a result, the CPU 37 integrally controls the respective units of the computer. The CPU 37 is an example of a "processor" according to the technology of the present disclosure. It should be noted that the memory 36 may be built in the CPU 37. The communication unit 38 performs transmission control of various types of information to an external device such as a device that performs the gene expression analysis 21.

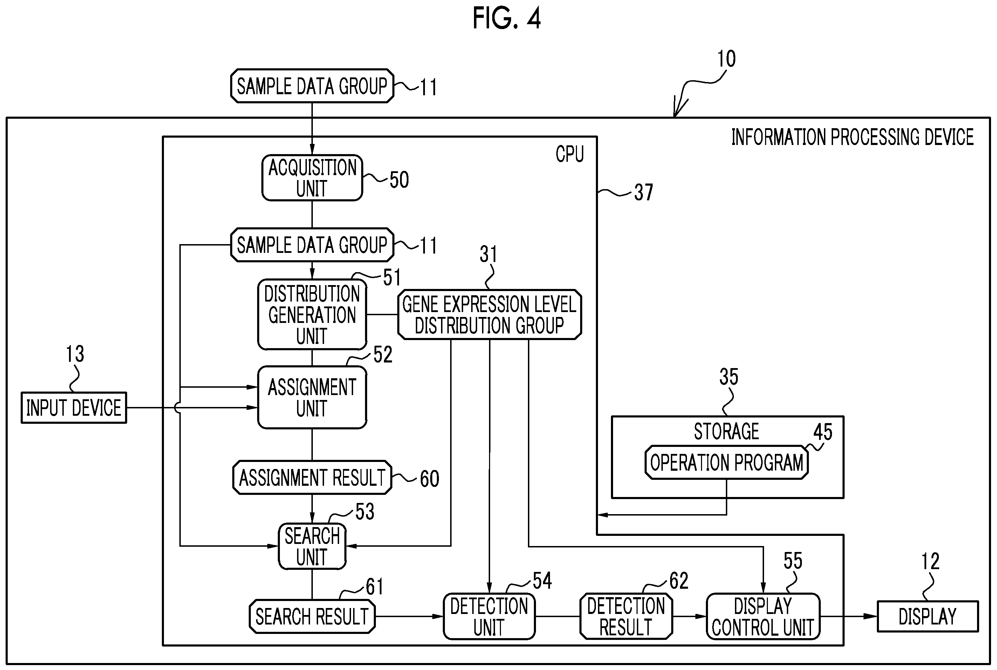

As shown in Fig.4 as an example, an operation program 45 is stored in the storage 35 of the information processing device 10. The operation program 45 is an application program for causing the computer to function as the information processing device 10. That is, the operation program 45 is an example of an "operation program of an information processing device" according to the technology of the present disclosure.

In a case in which the operation program 45 is activated, the CPU 37 of the computer constituting the information processing device 10 functions as an acquisition unit 50, a distribution generation unit 51, an assignment unit 52, a search unit 53, a detection unit 54, and a display control unit 55 in cooperation with the memory 36 and the like.

The acquisition unit 50 acquires the sample data group 11. The sample data group 11 is output from the acquisition unit 50 to the distribution generation unit 51, the assignment unit 52, and the search unit 53. More specifically, the sample data group 11 is once stored in the storage 35, and then is read out from the storage 35 as appropriate and output to the distribution generation unit 51, the assignment unit 52, and the search unit 53.

As shown in Fig. 2, the distribution generation unit 51 generates the gene expression level distribution group 31 based on the sample data group 11. The gene expression level distribution group 31 is output from the distribution generation unit 51 to the search unit 53, the detection unit 54, and the display control unit 55. Similarly to the sample data group 11, the gene expression level distribution group 31 is once stored in the storage 35, and then is read out from the storage 35 as appropriate and output to the search unit 53, the detection unit 54, and the display control unit 55.

The assignment unit 52 assigns a cluster to which each sample i of the high-expression group HG and the low-expression group LG is estimated to belong in the gene expression level distribution 30, to each sample i, for each of the plurality of candidate genes. The assignment unit 52 generates an assignment result 60 of the clusters, and outputs the generated assignment result 60 to the search unit 53.

The search unit 53 searches for a probability distribution P that fits the gene expression level distributions 30 of the high-expression group HG and the low-expression group LG for each of the plurality of candidate genes, based on the assignment result 60. The search unit 53 generates a search result 61 of the probability distribution P, and outputs the generated search result 61 to the detection unit 54.

The detection unit 54 detects the DEG from among the plurality of candidate genes based on the search result 61. The detection unit 54 generates a detection result 62 of the DEG, and outputs the generated detection result 62 to the display control unit 55. It should be noted that, as in the sample data group 11 and the like, the assignment result 60, the search result 61, and the detection result 62 are also once stored in the storage 35, and then are read out from the storage 35 as appropriate and output to the search unit 53, the detection unit 54, and the display control unit 55.

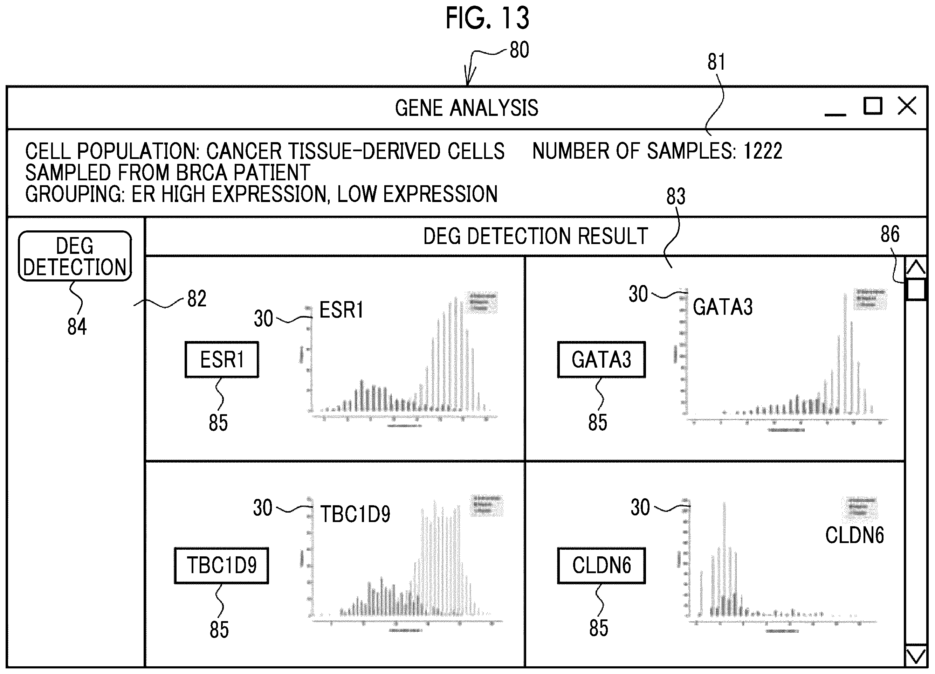

The display control unit 55 controls the display of the various screens on the display 12. The various screens include a gene analysis screen 80 (see Fig. 13) on which the detection result 62 is displayed.

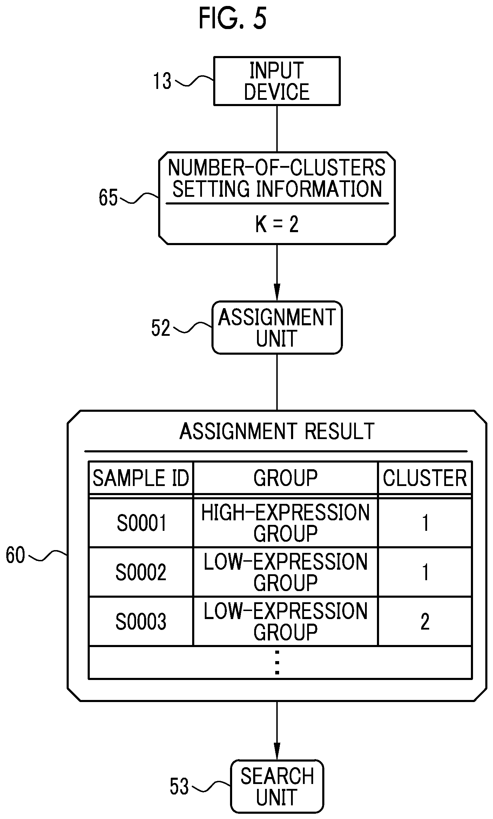

As shown in Fig. 5 as an example, number-of-clusters setting information 65 is input to the assignment unit 52. The number-of-clusters setting information 65 includes a number-of-clusters setting value K. The number-of-clusters setting value K is input by the user 14 through the input device 13. The user 14 inputs the number of the clusters estimated to be latent in the gene expression level distribution 30 as the number-of-clusters setting value K. In the gene expression level distribution 30 related to the cancer tissue-derived cells 20 of the BRCA patient 18 of the present example, it is estimated that two clusters are present in accordance with the gene HER2, based on the findings. Therefore, in the present example, the number-of-clusters setting value K = 2 is set.

The assignment unit 52 assigns any one of the clusters $z_i \in \{1,2\}$ to each sample i based on the gene expression level of the gene HER2, and generates the assignment result 60. In the assignment result 60, the group (high-expression group HG or low-expression group LG) to which the sample i belongs and the assigned cluster (cluster 1 or cluster 2) are registered for each sample ID.

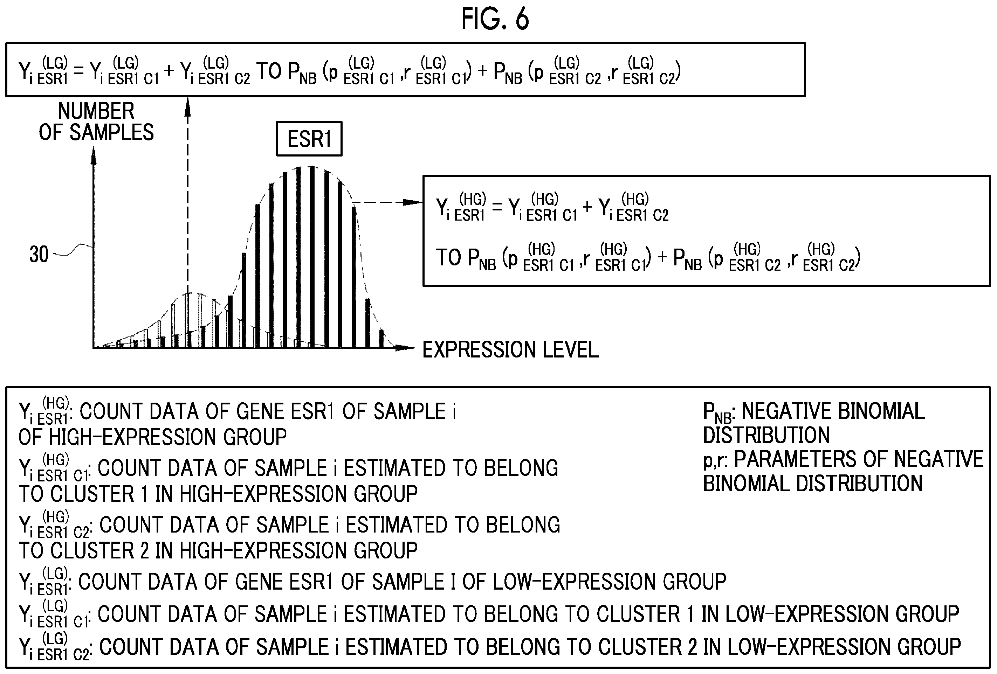

As shown in Fig. 6 as an example, count data $Y_{iESR1}^{(HG)}$ of the gene ESR1 of the sample i of the high-expression group HG is represented by Expression (1).

$$Y_{iESR1}^{(HG)} = Y_{iESR1C1}^{(HG)} + Y_{iESR1C2}^{(HG)} \dots (1)$$

Here, $Y_{iESR1C1}^{(HG)}$ is count data of the sample i estimated to belong to the cluster 1 in the high-expression group HG, and $Y_{iESR1C2}^{(HG)}$ is count data of the sample i estimated to belong to the cluster 2 in the high-expression group HG.

Similarly, count data $Y_{iESR1}^{(LG)}$ of the gene ESR1 of the sample i of the low-expression group LG is represented by Expression (2).

$$Y_{iESR1}^{(LG)} = Y_{iESR1C1}^{(LG)} + Y_{iESR1C2}^{(LG)} \dots (2)$$

Here, $Y_{iESR1C1}^{(LG)}$ is count data of the sample i estimated to belong to the cluster 1 in the low-expression group LG, and $Y_{iESR1C2}^{(LG)}$ is count data of the sample i estimated to belong to the cluster 2 in the low-expression group LG.

In a case in which Expressions (1) and (2) are more generally expressed, the count data $Y_{ig}^{(j)}$ of the gene g of the sample i of the group j is represented by Expression (3).

$$Y_{ig}^{(j)} = \sum_{k=1}^{K} \left( Y_{igk}^{(j)} \delta_{z_i, k=1} \right) \cdots (3)$$

It should be noted that $\delta_{z_{ik}}=1$ is a Kronecker delta.

The search unit 53 searches for the probability distribution P that fits the gene expression level distribution 30 based on the assumption that the gene expression level distribution 30 follows the probability distribution P having the parameter $\lambda$. The search unit 53 searches for the probability distribution P for each cluster. That is, the search unit 53 searches for the probability distribution $P(p_{gk}, r_{gk})$ on the assumption that the gene expression level distribution 30 of the gene g related to the population of the samples i to which the clusters k are assigned follows the probability distribution $P(p_{gk}, r_{gk})$ having the parameter $\lambda_k = (p_{gk}, r_{gk})$. It should be noted that $p_{gk}$ and $r_{gk}$ are numerical values that determine the shape of the probability distribution P, and are, for example, the mean, the variance, and the like.

Here, it is known that each count data in the gene expression level distribution 30 well follows a negative binomial distribution $P_{NB}(p,r)$, which is one type of probability distribution P. Therefore, the count data $Y_{iESR1}^{(HG)}$ of the gene ESR1 of the sample i of the high-expression group HG is further represented by Expression (4).

$$Y_{iESR1}^{(HG)} = Y_{iESR1C1}^{(HG)} + Y_{iESR1C2}^{(HG)} \sim P_{NB}(p_{ESR1C1}^{(HG)}, r_{ESR1C1}^{(HG)}) + P_{NB}(p_{ESR1C2}^{(HG)}, r_{ESR1C2}^{(HG)}) \dots (4) \quad (4)$$

Similarly, the count data $Y_{iESR1}^{(LG)}$ of the gene ESR1 of the sample i of the low-expression group LG is further represented by Expression (5).

$$Y_{iESR1}^{(LG)} = Y_{iESR1C1}^{(LG)} + Y_{iESR1C2}^{(LG)} \sim P_{NB}(p_{ESR1C1}^{(LG)}, r_{ESR1C1}^{(LG)}) + P_{NB}(p_{ESR1C2}^{(LG)}, r_{ESR1C2}^{(LG)}) \dots (5) \quad (5)$$

In a case in which Expressions (4) and (5) are more generally expressed, the count data $Y_{ig}^{(j)}$ of the gene g of the sample i of the group j can be further expressed by Expression (6).

$$Y_{ig}^{(j)} \sim \sum_{k=1}^{K} \{ P_{NB}(p_{gk}^{(j)}, r_{gk}^{(j)}) \, \delta_{z_i k=1} \} \cdots (6)$$

It should be noted that the negative binomial distribution $P_{NB}(p,r)$ is an example of a "first probability distribution" according to the technology of the present disclosure.

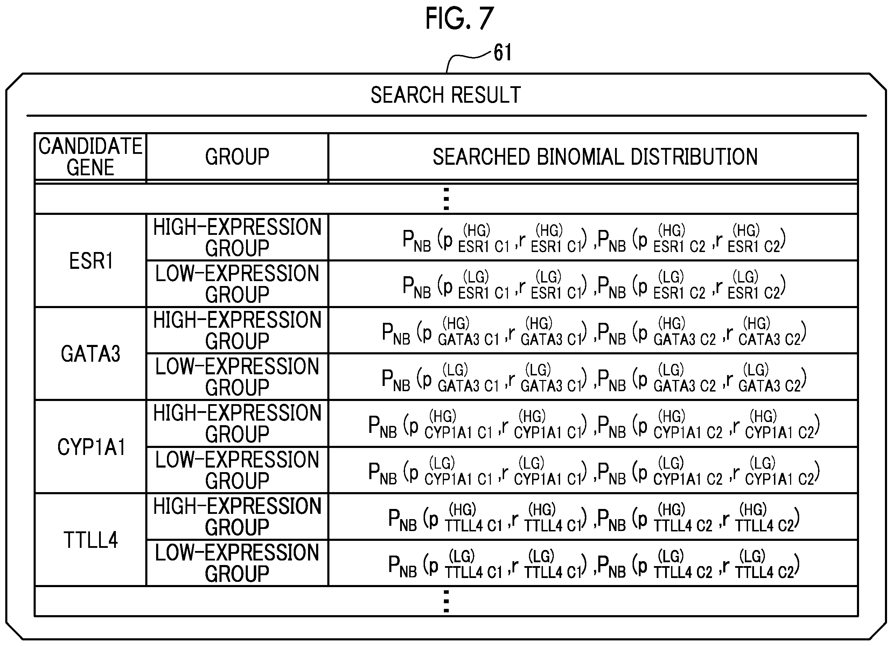

The search unit 53 optimizes the parameters p and r of the negative binomial distribution $P_{NB}(p,r)$ of each cluster to fit well to the gene expression level distribution 30, using, for example, a variational Bayesian method or a Markov chain Monte Carlo method. As shown in Fig. 7 as an example, the search result 61 is data in which the negative binomial distribution $P_{NB}(p,r)$ of each cluster in which the parameters p and r are optimized in this way is registered for each of the plurality of candidate genes and for each of the high-expression group HG and the low-expression group LG.

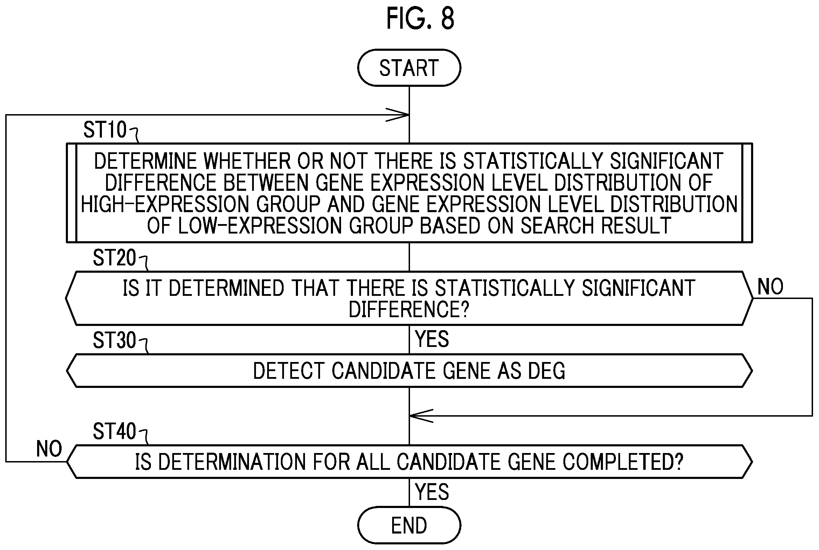

As shown in the flowchart of Fig. 8 as an example, the detection unit 54 detects the DEG in accordance with the following procedure. First, the detection unit 54 determines whether or not there is a statistically significant difference between the gene expression level distribution 30 of the high-expression group HG and the gene expression level distribution 30 of the low-expression group LG for one candidate gene based on the search result 61 of the negative binomial distribution $P_{NB}(p,r)$ (step ST10).

In a case in which it is determined that there is the statistically significant difference (YES in step ST20), the detection unit 54 detects the candidate gene as the DEG (step ST30). On the other hand, in a case in which it is determined that there is no statistically significant difference (NO in step ST20), the detection unit 54 does not detect the candidate gene as the DEG. The detection unit 54 repeats the processing of step ST10, step ST20, and step ST30 until the determination for all the candidate genes is completed (NO in step ST40).

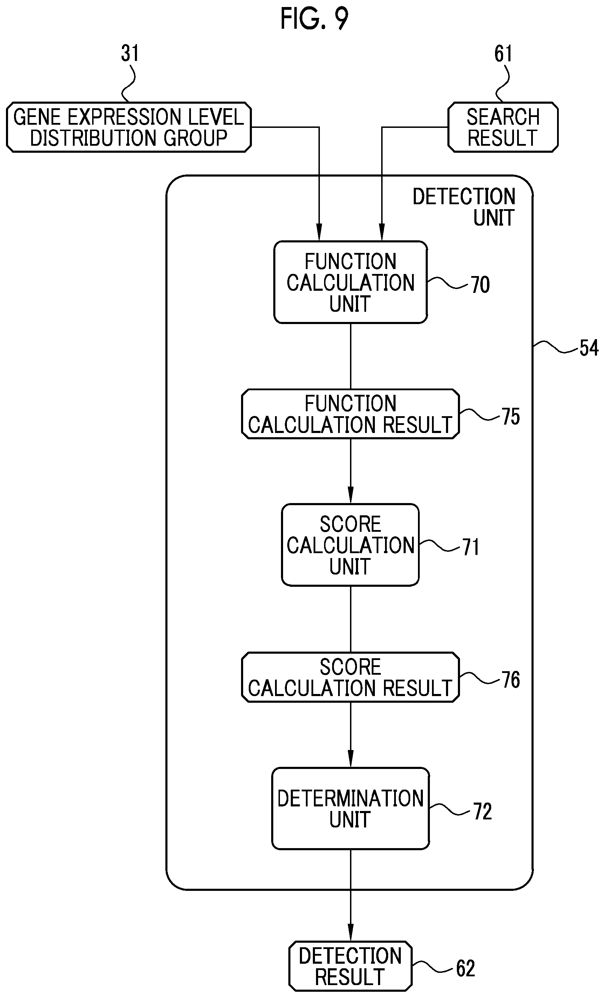

As shown in Fig. 9 as an example, the detection unit 54 includes a function calculation unit 70, a score calculation unit 71, and a determination unit 72.

The gene expression level distribution group 31 and the search result 61 are input to the function calculation unit 70. The function calculation unit 70 calculates a logarithmic likelihood function $l_g^{(j)}$ for each of the high-expression group HG and the low-expression group LG from the gene expression level distribution group 31 and the search result 61. Further, the function calculation unit 70 calculates a logarithmic likelihood function $l_g$ of all samples i in both groups of the high-expression group HG and the low-expression group LG. The function calculation unit 70 outputs a calculation result (hereinafter, referred to as a function calculation result) 75 of the logarithmic likelihood function $l_g^{(j)}$ and the logarithmic likelihood function $l_g$ to the score calculation unit 71.

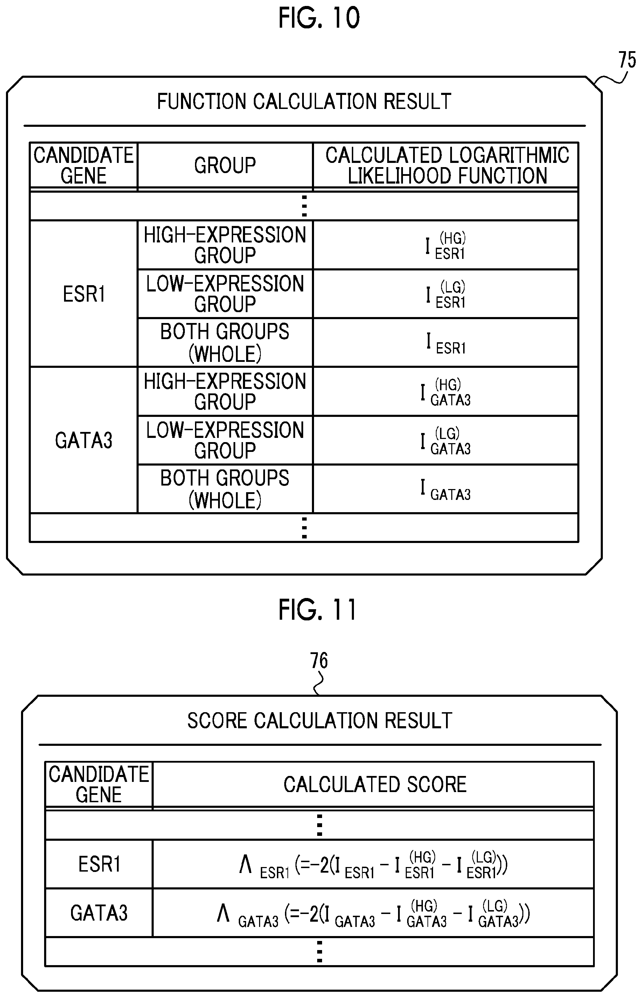

As shown in Fig. 10 as an example, the function calculation result 75 is data in which the calculated logarithmic likelihood function $l_g^{(HG)}$ of the high-expression group HG, the calculated logarithmic likelihood function $l_g^{(LG)}$ of the low-expression group LG, and the calculated logarithmic likelihood function $l_g$ of the entire group are registered for each of the plurality of candidate genes. The logarithmic likelihood function $l_g^{(j)}$ of the high-expression group HG and the low-expression group LG is an example of a "representative index value" according to the technology of the present disclosure. It should be noted that the representative index value may be an average value of the gene expression level distributions 30 of the high-expression group HG and the low-expression group LG.

The score calculation unit 71 calculates a score $\Lambda_g$ representing a degree of difference between the high-expression group HG and the low-expression group LG from the function calculation result 75. The score calculation unit 71 outputs a calculation result (hereinafter, referred to as a score calculation result) 76 of the score $\Lambda_g$ to the determination unit 72.

As shown in Fig. 11 as an example, the score calculation result 76 is data in which the calculated score $\Lambda_g$ is registered for each of the plurality of candidate genes. It should be noted that the score $\Lambda_g$ is obtained by calculating Expression (7).

$$\Lambda_g = -2(l_g - l_g^{(HG)} - l_g^{(LG)}) \ldots (7)$$

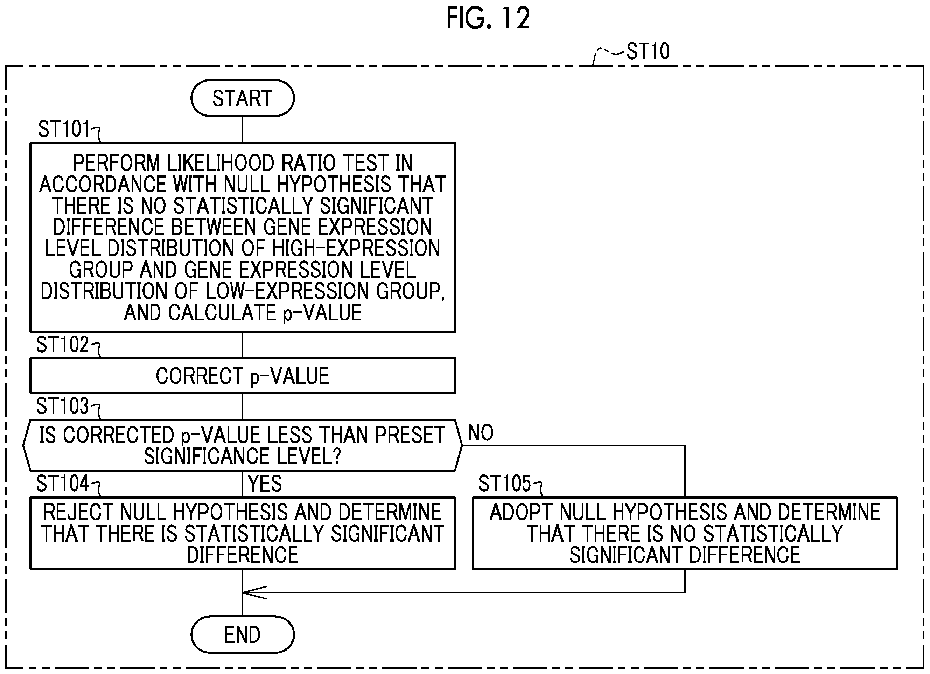

The determination unit 72 executes processing of step ST10 of the flowchart shown in Fig. 8. More specifically, as shown in the flowchart of Fig. 12, the determination unit 72 performs a likelihood ratio test in accordance with the null hypothesis that there is no statistically significant difference between the gene expression level distribution 30 of the high-expression group HG and the gene expression level distribution 30 of the low-expression group LG, using the score $\Lambda_g$, and calculates a p-value (step ST101). The likelihood ratio test tests how much the score $\Lambda_g$ follows the $\chi^2$ distribution of the degree of freedom $\theta = 2K + 1$.

Then, the determination unit 72 corrects the p-value by, for example, Bonferroni correction (step ST102). This correction is performed in order to solve a problem of multiple tests that may occur in a case in which the likelihood ratio test is applied to a target having a large number of the degrees of freedom, such as a gene.

In a case in which the p-value after the correction is less than a preset significance level (YES in step ST103), the determination unit 72 rejects the null hypothesis established in step ST101, and determines that there is the statistically significant difference between the gene expression level distribution 30 of the high-expression group HG and the gene expression level distribution 30 of the low-expression group LG (step ST104). In such a case, the determination unit 72 outputs the detection result 62 in which the gene is the DEG.

On the other hand, in a case in which the corrected p-value is equal to or greater than the preset significance level (NO in step ST103), the determination unit 72 adopts the null hypothesis established in step ST101 and determines that there is no statistically significant difference between the gene expression level distribution 30 of the high-expression group HG and the gene expression level distribution 30 of the low-expression group LG (step ST105). In such a case, the determination unit 72 does not detect the gene as the DEG. It should be noted that the significance level is, for example, 0.05.

As shown in Fig. 13 as an example, the gene analysis screen 80 displayed on the display 12 under the control of the display control unit 55 includes a first display region 81, a second display region 82, and a third display region 83. In the first display region 81, basic information on the target of the gene analysis, such as the cell population, the number of the samples, and grouping, is displayed. A DEG detection button 84 is disposed in the second display region 82. In a case in which the DEG detection button 84 is selected, each processing unit of the CPU 37 operates to detect the DEG.

Nothing is displayed in the third display region 83 before the DEG detection button 84 is selected. On the other hand, in a case in which the DEG detection button 84 is selected and the detection of the DEG is performed, the display control unit 55 displays, as shown, a set of a flag 85 indicating the gene detected as the DEG and the gene expression level distributions 30 of the gene detected as the DEG in the third display region 83 in a list. A scroll bar 86 is provided in the third display region 83, and the third display region 83 can be scrolled and displayed up and down. The user 14 can check the DEG from the display in the third display region 83.

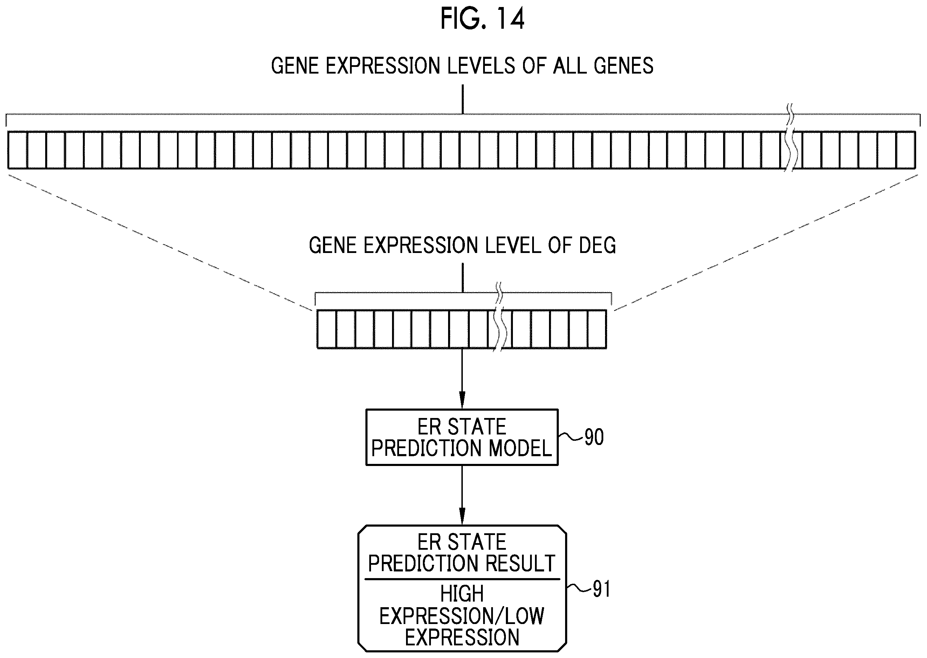

As shown in Fig. 14 as an example, the DEG detected as described above is used, for example, in a case of selecting input data of the ER state prediction model 90. The ER state prediction model 90 is a machine learning model in which the gene expression level of the DEG of the cancer tissue-derived cells 20 sampled from the breast 19 of the BRCA patient 18 in which whether ER is highly expressed or lowly expressed is unknown is used as the input data, and an ER state prediction result 91 is used as output data. The ER state prediction result 91 is any one of "high expression" in a case in which it is predicted that the ER is highly expressed or "low expression" in a case in which it is predicted that the ER is low expression. In this way, by narrowing down the input data from the gene expression levels of all genes to the gene expression level of the DEG, the prediction accuracy of the ER state prediction result 91 can be further improved.

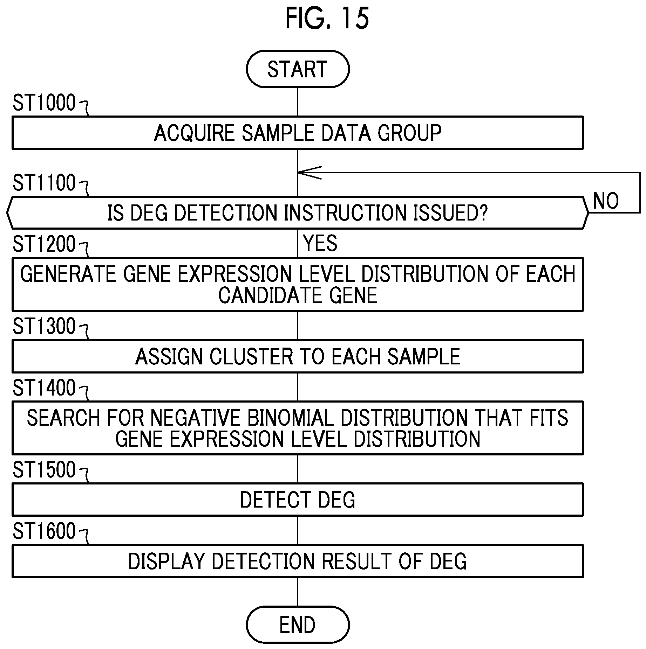

Next, an operation of the configuration described above will be described with reference to the flowchart shown in Fig. 15 as an example. In a case in which the operation program 45 is activated in the information processing device 10, as shown in Fig. 4, the CPU 37 of the information processing device 10 functions as the acquisition unit 50, the distribution generation unit 51, the assignment unit 52, the search unit 53, the detection unit 54, and the display control unit 55.

First, the acquisition unit 50 acquires the sample data group 11 (step ST1000). The sample data group 11 is output from the acquisition unit 50 to the distribution generation unit 51, the assignment unit 52, and the search unit 53.

The gene analysis screen 80 is displayed on the display 12 under the control of the display control unit 55. In a case in which the DEG detection button 84 is selected by the user 14 on the gene analysis screen 80 (YES in the step ST1100), the gene expression level distribution 30 of each candidate gene is generated in the distribution generation unit 51 based on the sample data group 11 as shown in Fig. 2 (step ST1200). The gene expression level distribution group 31, which is a collection of the gene expression level distributions 30 of the respective candidate genes, is output from the distribution generation unit 51 to the search unit 53, the detection unit 54, and the display control unit 55.

As shown in Fig. 5, in the assignment unit 52, the cluster is assigned to each sample i (step ST1300). The assignment result 60 of the clusters is output from the assignment unit 52 to the search unit 53.

Then, as shown in Figs. 6 and 7, in the search unit 53, the negative binomial distribution $P_{NB}(p,r)$ that fits the gene expression level distribution 30 is searched for, for each cluster (step ST1400). The search result 61 of the negative binomial distribution $P_{NB}(p,r)$ is output from the search unit 53 to the detection unit 54.

Then, as shown in Figs. 8 to 12, in the detection unit 54, the DEG is detected (step ST1500). The detection result 62 of the DEG is output from the detection unit 54 to the display control unit 55.

Finally, as shown in Fig. 13, under the control of the display control unit 55, the set of the flag 85 indicating the gene detected as the DEG and the gene expression level distribution 30 of the gene detected as the DEG is displayed in a list in the third display region 83 of the gene analysis screen 80 (step ST1600). As a result, the detection result 62 of the DEG is available for viewing by the user 14.

As described above, the CPU 37 of the information processing device 10 comprises the assignment unit 52 and the search unit 53. The assignment unit 52 assigns a cluster to which each sample i of the high-expression group HG and the low-expression group LG, in which the cancer tissue-derived cell population 24 is divided in accordance with the ER state information 16, is estimated to belong in the gene expression level distribution 30, to each sample i, for each of a plurality of candidate genes which are the candidates for the DEG. The search unit 53 searches for the negative binomial distribution $P_{NB}(p,r)$ that fits the gene expression level distributions 30 of the high-expression group HG and the low-expression group LG for each of the plurality of candidate genes, based on the assignment result 60 of the clusters.

Therefore, it is possible to search for the negative binomial distribution $P_{NB}(p,r)$ that well fits the gene expression level distribution 30 of the cancer tissue-derived cell population 24 in which the plurality of subtypes are mixed and a plurality of clusters due to the plurality of subtypes are expected to appear. As a result, it is possible to reduce the probability of making an error in the determination of whether or not there is the statistically significant difference between the gene expression level distribution 30 of the high-expression group HG and the gene expression level distribution 30 of the low-expression group LG. Therefore, even in a case in which the cell population in which the plurality of subtypes are mixed, such as the cancer tissue-derived cell population 24, is an analysis target, it is possible to detect an appropriate DEG.

The detection unit 54 determines whether or not there is the statistically significant difference between the gene expression level distribution 30 of the high-expression group HG and the gene expression level distribution 30 of the low-expression group LG for each of the plurality of candidate genes based on the search result 61 of the negative binomial distribution $P_{NB}(p,r)$. The detection unit 54 detects the candidate gene for which it is determined that there is the statistically significant difference between the gene expression level distribution 30 of the high-expression group HG and the gene expression level distribution 30 of the low-expression group LG, as the DEG. Therefore, it is possible to detect the gene that is plausible and convincing as the DEG.

The determination unit 72 of the detection unit 54 calculates the logarithmic likelihood function $l_g^{(j)}$ for each of the high-expression group HG and the low-expression group LG from the gene expression level distribution 30 and the searched negative binomial distribution $P_{NB}(p,r)$. The determination unit 72 calculates the score $\Lambda_g$ representing the degree of difference between the high-expression group HG and the low-expression group LG from the logarithmic likelihood function $l_g^{(j)}$, and performs the determination based on the score $\Lambda_g$. Therefore, it is possible to detect the gene that is more plausible and convincing as the DEG.

The first probability distribution searched for by the search unit 53 is the negative binomial distribution $P_{NB}(p,r)$. Therefore, it is possible to detect the DEG that coincides with the finding that the gene expression level distribution 30 follows the negative binomial distribution $P_{NB}(p,r)$. It should be noted that the first probability distribution is not limited to the negative binomial distribution $P_{NB}(p,r)$ as the example. A binomial distribution, a Poisson distribution, and the like may be used.

[Second Embodiment]

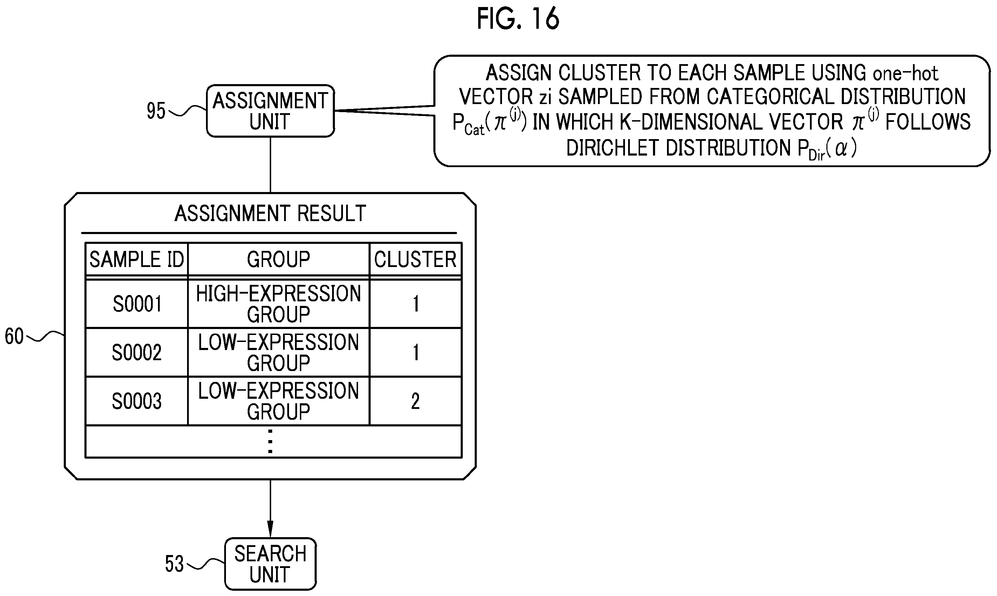

In the first embodiment, the cluster is assigned to each sample i based on the gene expression level of the gene HER2, but the present disclosure is not limited to this. As an example, as in the assignment unit 95 shown in Fig. 16, the cluster may be assigned to each sample i by a one-hot vector $z_i$ sampled from a categorical distribution $P_{cat}(\pi^{(j)})$. The categorical distribution $P_{cat}(\pi^{(j)})$ is a distribution in which a K-dimensional vector $\pi^{(j)}$ follows a Dirichlet distribution $P_{Dir}(\alpha)$ having the parameter $\alpha_k = 1, 2, ..., K$. As can be seen from the subscript (j), the categorical distribution $P_{cat}(\pi^{(j)})$ is prepared for each of the high-expression group HG and the low-expression group LG. The categorical distribution $P_{cat}(\pi^{(j)})$ is an example of a "second probability distribution" according to the technology of the present disclosure.

As described above, in the second embodiment, the assignment unit 95 performs the assignment of the clusters by using the categorical distribution $P_{cat}(\pi^{(j)})$. Therefore, even in a case in which there is no prior knowledge about the clusters, the assignment of the clusters can be performed as in the gene expression level distribution 30 related to the cancer tissue-derived cells 20 sampled from the BRCA patient 18 of the first embodiment. It should be noted that the second probability distribution is not limited to the categorical distribution $P_{cat}(\pi^{(j)})$, and may be, for example, a multinomial distribution. In addition, in a case in which there is no prior knowledge about the clusters, the clusters may be randomly assigned instead of using the second probability distribution such as the categorical distribution $P_{cat}(\pi^{(j)})$. Further, a nonparametric method such as a k-nearest neighbor method may be used to assign the clusters.

[Third Embodiment]

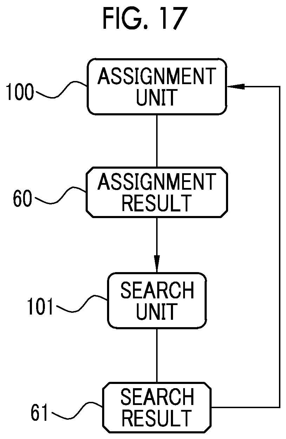

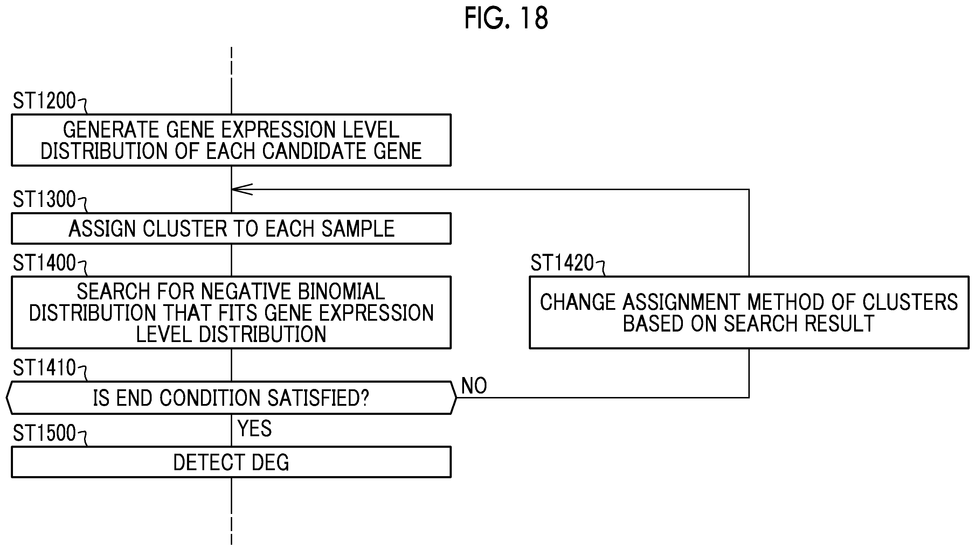

As shown in Figs. 17 and 18 as an example, in the third embodiment, the search result 61 from the search unit 101 is fed back to the assignment unit 100. The assignment unit 100 changes the assignment method of the clusters based on the search result 61 fed back from the search unit 101 (step ST1420), and re-assigns the clusters using the changed assignment method (step ST1300). The search unit 101 re-searches for the negative binomial distribution $P_{NB}(p,r)$ that fits the gene expression level distribution 30 based on a re-assignment result 60 of the clusters (a step ST1400). While the end condition is not satisfied (NO in step ST1410), the assignment unit 100 and the search unit 101 repeat the processing of re-assigning the clusters and re-searching for the negative binomial distribution $P_{NB}(p,r)$.

The change in the assignment method of the clusters in step ST1420 is performed, for example, in a case of the assignment unit 95 according to the second embodiment, by changing the parameter $\pi^{(j)}$ of the categorical distribution $P_{cat}$

$(\pi^{(j)})$ based on the search result 61. In addition, in step ST1410, for example, in a case in which a difference between the parameters p and r of the negative binomial distribution $P_{NB}(p,r)$ previously searched for and the negative binomial distribution $P_{NB}(p,r)$ currently searched for is less than a threshold value set in advance, it is determined that an end condition is satisfied. Alternatively, the end condition may be satisfied in a case in which the processing is repeated a preset number of times.

As described above, in the third embodiment, the assignment unit 100 and the search unit 101 perform, at least once, the processing of re-assigning the clusters by receiving the search result 61 of the negative binomial distribution $P_{NB}(p,r)$ and re-searching for the negative binomial distribution $P_{NB}(p,r)$ based on the re-assignment result 60 of the clusters. In a case in which such processing is performed at least once, the assignment method of the clusters and the search of the negative binomial distribution $P_{NB}(p,r)$ are refined, and the negative binomial distribution $P_{NB}(p,r)$ that more fits the gene expression level distribution 30 can be searched for. Therefore, it is possible to further reduce the probability of making an error in the determination of whether or not there is the statistically significant difference between the gene expression level distribution 30 of the high-expression group HG and the gene expression level distribution 30 of the low-expression group LG.

[Fourth Embodiment]

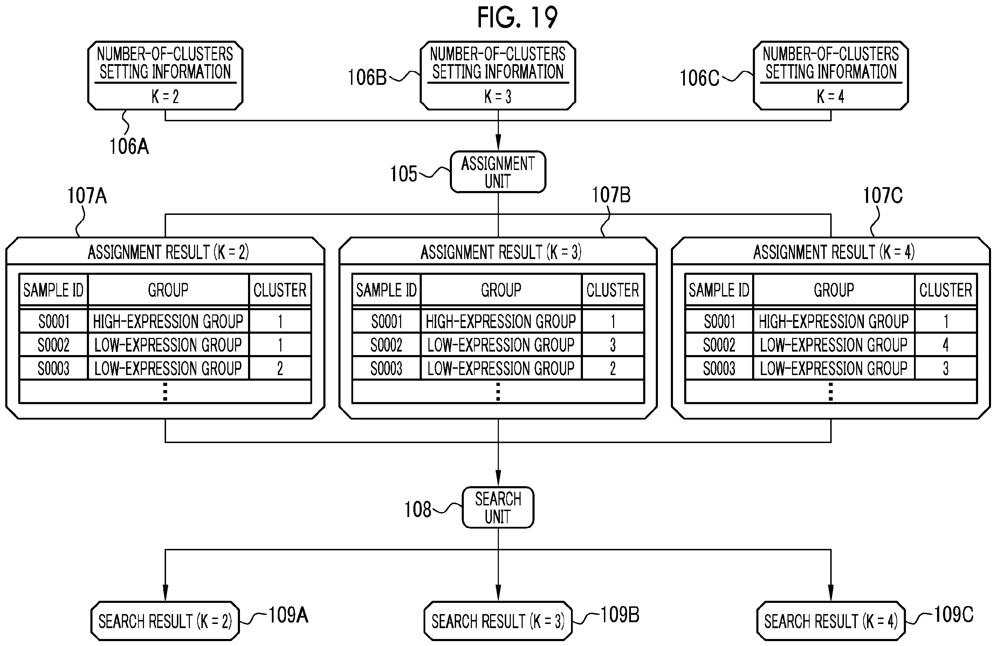

In the first embodiment, the number-of-clusters setting value K is set to one type, but the present disclosure is not limited to this. As an example, as in the assignment unit 105 shown in Fig. 19, the input of number-of- clusters setting information 106A, 106B, and 106C including three types of number-of-clusters setting values (K = 2, K = 3, and K = 4) may be received. The three types of number-of-clusters setting values may be input by the user 14 via the input device 13, or may be automatically input by the CPU 37.

The assignment unit 105 assigns the cluster to each sample i based on each of the three types of number-of-clusters setting values, and outputs assignment results 107A, 107B, and 107C. The assignment result 107A corresponds to the number-of-clusters setting value K = 2, the assignment result 107B corresponds to the number-of-clusters setting value K = 3, and the assignment result 107C corresponds to the number-of-clusters setting value K = 4.

The search unit 108 searches for the negative binomial distribution $P_{NB}(p,r)$ based on the assignment result 107A, and outputs a search result 109A. In addition, the search unit 108 searches for the negative binomial distribution $P_{NB}(p,r)$ based on the assignment result 107B, and outputs a search result 109B. Further, the search unit 108 searches for the negative binomial distribution $P_{NB}(p,r)$ based on the assignment result 107C, and outputs a search result 109C.

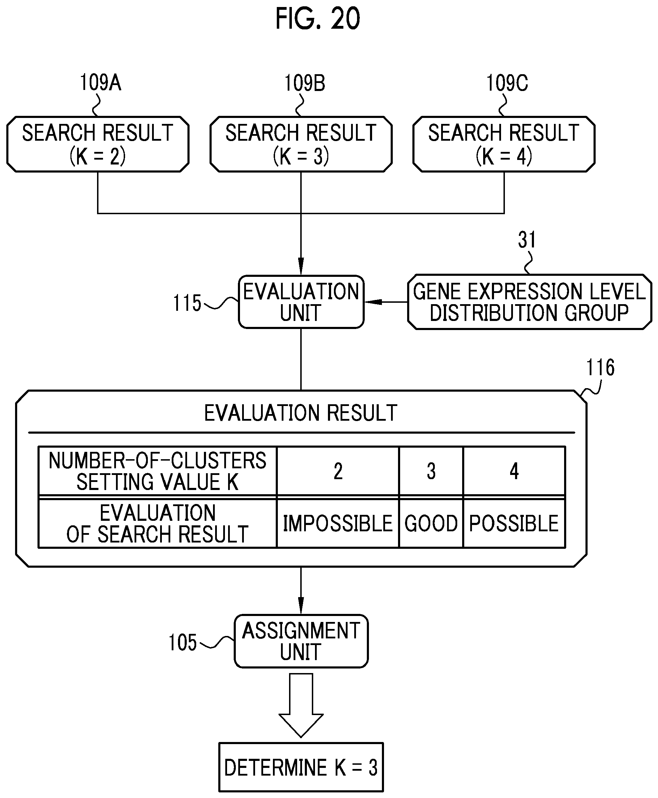

As shown in Fig. 20 as an example, the CPU of the information processing device according to the fourth embodiment includes an evaluation unit 115. The search results 109A, 109B, and 109C from the search unit 108 are input to the evaluation unit 115. Further, the gene expression level distribution group 31 from the distribution generation unit 51 is input to the evaluation unit 115. The evaluation unit 115 evaluates how much the negative binomial distributions $P_{NB}(p,r)$ of the search results 109A, 109B, and 109C fit the gene expression level distribution 30 by using information amount criterion, for example, a widely applicable information criterion (WAIC) and outputs an evaluation result 116 to the assignment unit 105.

The assignment unit 105 determines the number-of-clusters setting value K at which the negative binomial distribution $P_{NB}(p,r)$ that best fits the gene expression level distribution 30 can be searched for based on the evaluation result 116. Fig. 20 shows an example in which the number-of-clusters setting value K = 3 is determined such that the evaluation result of the search result 109A in a case in which the number-of-clusters setting value K = 2 is "not possible", the evaluation result of the search result 109B in a case in which the number-of-clusters setting value K = 3 is "good", and the evaluation result of the search result 109C in a case in which the number-of-clusters setting value K = 4 is "possible".

As described above, in the fourth embodiment, the assignment unit 105 changes the number of the clusters to be assigned. The search unit 108 searches for the negative binomial distribution $P_{NB}(p,r)$ for each changed number. The assignment unit 105 can determine a number in which the negative binomial distribution $P_{NB}(p,r)$ that best fits the gene expression level distribution 30 is searchable for from among the changed numbers, based on the search results 109A, 109B, and 109C of the negative binomial distribution $P_{NB}(p,r)$. Therefore, even in a case in which there is no knowledge about the number of the clusters unlike the first embodiment, an appropriate number of the clusters can be set. Therefore, it is possible to further reduce the probability of making an error in the determination of whether or not there is the statistically significant difference between the gene expression level distribution 30 of the high-expression group HG and the gene expression level distribution 30 of the low-expression group LG.

It should be noted that the number of the clusters to be changed is not limited to the three types as an example. For example, K may be nine types of 2, 3, 4, ..., 8, 9, and 10, or K may be two types of 2 and 3. In a case in which the evaluation result is "good" with the number of the two or more types of clusters, it is preferable to determine the number of the clusters to be smaller in consideration of simplification of processing. In addition, the fourth embodiment may be carried out in combination with the second embodiment and/or the third embodiment.

[5_1St Embodiment]

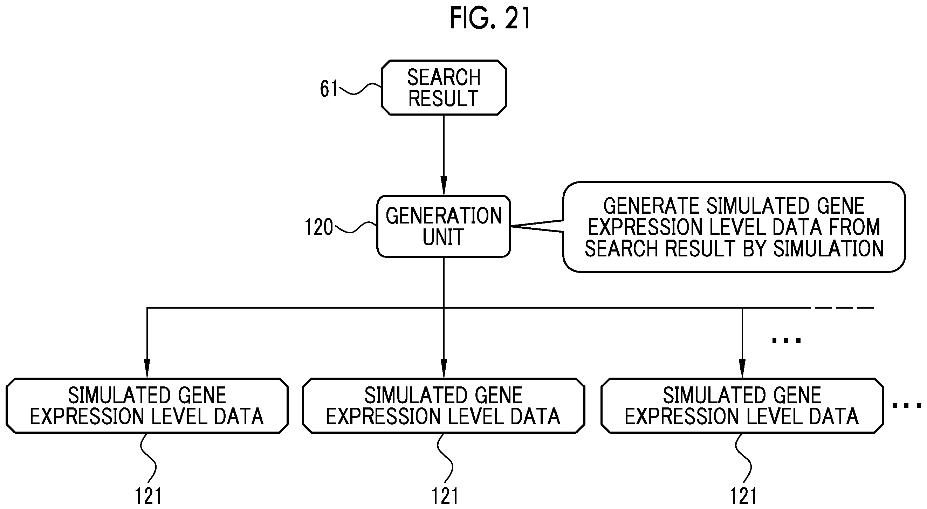

As shown in Fig. 21 as an example, the CPU of the information processing device of the 5_1st embodiment includes a generation unit 120. The search result 61 from the search unit 53 is input to the generation unit 120. The generation unit 120 generates simulated gene expression level data (hereinafter, referred to as simulated gene expression level data) 121 from the search result 61 by simulation. Theoretically, the generation unit 120 can generate the simulated gene expression level data 121 without any limit on the number. Here, the simulation is processing of performing the series of processing of generating the gene expression level distribution 30 from the gene expression level data 17, assigning the cluster to each sample i, and searching for the negative binomial distribution $P_{NB}(p,r)$ that fits the gene expression level distribution 30 based on the assignment result 60, in a backward manner.

As described above, in the 5_1st embodiment, the generation unit 120 generates the simulated gene expression level data 121 by the simulation based on the searched negative binomial distribution $P_{NB}(p,r)$. The characteristics of the cancer tissue-derived cell population 24 can be clarified by analyzing the simulated gene expression level data 121. In addition, by calculating an average value of the gene expression levels of the simulated gene expression level data 121 and comparing the calculated average value with the gene expression level of the gene expression level data 17, it is possible to extract an abnormal value of the gene expression level due to measurement noise or the like, which is hidden in the gene expression level data 17. As a result, it is possible to search for the negative binomial distribution $P_{NB}(p,r)$ after excluding the extracted abnormal value from the gene expression level data 17, and thus it is possible to search for a more appropriate negative binomial distribution $P_{NB}(p,r)$.

[5_2Nd Embodiment]

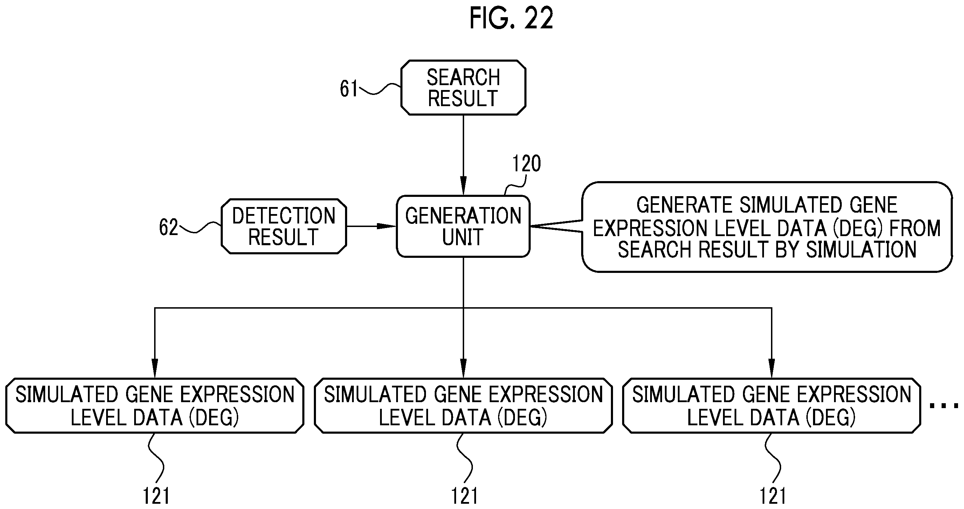

As shown in Fig. 22 as an example, in the 5_2nd embodiment, the generation unit 120 generates the simulated gene expression level data 121 only for the DEG. In such a case, in addition to the search result 61, a detection result 62 from the detection unit 54 is also input to the generation unit 120.

In this way, in the 5_2nd embodiment, the generation unit 120 generates only the simulated gene expression level data 121 of DEG by the simulation. By analyzing the simulated gene expression level data 121 of the DEG, it is possible to clarify a characteristic difference between the other genes and the DEG. In addition, it is possible to reduce the processing load of the simulation, as compared with the 5_1st embodiment in which the simulated gene expression level data 121 is generated for all the candidate genes.

The 5_1st embodiment in which the simulated gene expression level data 121 is generated for all the candidate genes and the 5_2nd embodiment in which the simulated gene expression level data 121 is generated only for the DEG may be selectable by the user 14. In addition, the gene for generating the simulated gene expression level data 121 may be designated by the user 14.

[Examples]

An example of the technology of the present disclosure will be described below. In the present example, the ER state information 16 of the BRCA patient 18 is acquired from the Genomic Data Commons (GDC) Legacy Archive managed by the National Cancer Institute (NCI). In addition, the gene expression level data 17 of the BRCA patient 18 is acquired from the cancer genome atlas (TCGA) operated by NCI. The gene expression level data 17 is data calculated by mapping RNA-Seq data obtained from the cancer tissue-derived cells 20 of the BRCA patient 18 to a reference genome by a high-throughput (HT) Seq module.

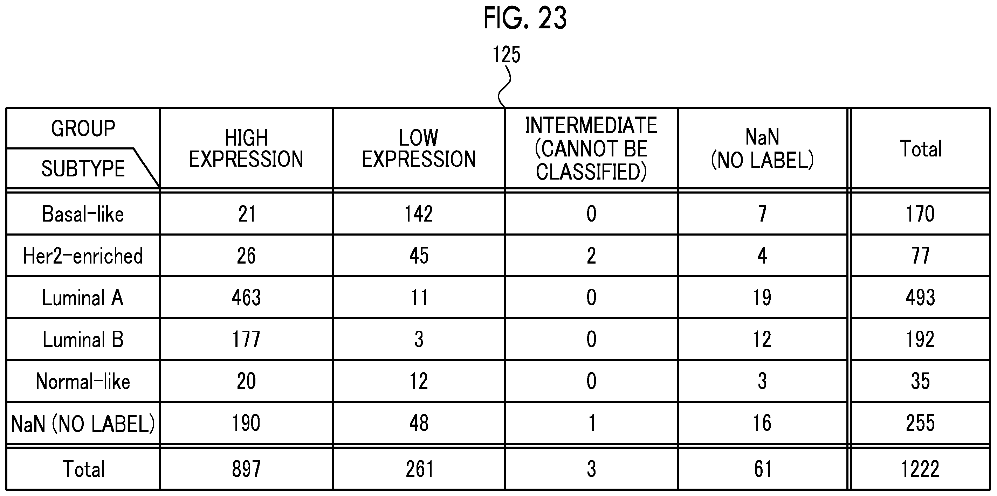

Table 125 of Fig. 23 shows a breakdown of the groups and the subtypes of the samples i in the present example. The number of the samples is 1222. Then, among the 1222 samples, the ER state information 16 is not added to 61 samples i, and the 61 samples i are not labeled (not a number (NaN)), and the ER state information 16 is added to the remaining 1161 samples i. In addition, the subtype information is not added to the 255 samples i, which are not labeled, and the subtype information is added to the remaining 967 samples i. Information provided by the TCGA Pan-Cancer Atlas project is used for this subtype information.

Among the genes for which the gene expression level is registered in the gene expression level data 17, 19140 genes encoding proteins are selected as the candidate genes. The number-of-clusters setting value K is set to 2 as in the first embodiment. The clusters are also assigned to the sample i having the subtype of "Normal-like" and the sample i having no label. A variational Bayesian method is used for searching for the negative binomial distribution $P_{NB}(p,r)$. In addition, as a comparative example, the DEG is detected using a related-art detection method described in Non-Patent Literature 1 (hereinafter, referred to as a related-art technology).

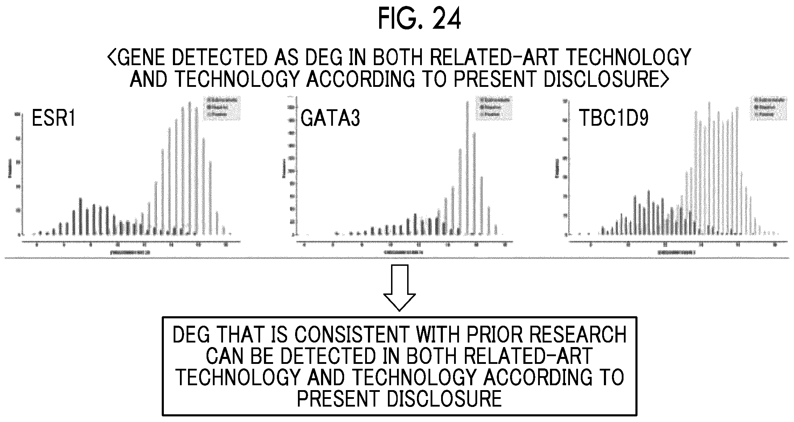

Fig. 24 shows the gene expression level distribution 30 of each of the genes ESR1, GATA3, and TBC1D9 detected as the DEGs in both the related-art technology and the technology of the present disclosure. These genes ESR1, GATA3, and TBC1D9 have the significant difference between the gene expression level distribution 30 of the high-expression group HG and the gene expression level distribution 30 of the low-expression group LG. In addition, the genes ESR1, GATA3, and TBC1D9 have already been reported as the DEGs in many literatures. In particular, the gene ESR1 is a gene involved in the production of ER$\alpha$. The gene GATA3 is recognized as a marker gene specific to the breast invasive cancer. In addition, the gene TBC1D9 has recently been reported as an important gene in a subtype "Basal-like" in, for example, the following literature A.

Literature A <C. Kothari, et al., "Tbc1d9: An important modulator of tumorigenesis in breast cancer." Cancers, 13 (14): 3557, 2021.>

As described above, both the related-art technology and the technology of the present disclosure can detect a gene that is consistent with the prior research as the DEG. Therefore, it can be said that the technology of the present disclosure is a method of detecting the DEG having validity comparable to that of the related-art technology.

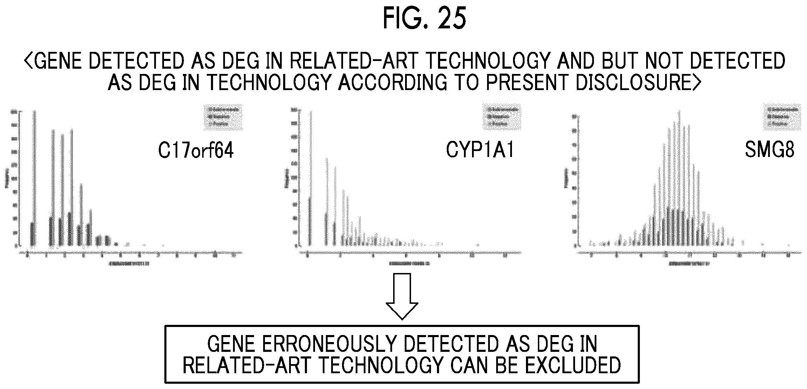

Fig. 25 shows the gene expression level distribution 30 of each of the genes C17orf64, CYP1A1, and SMG8 that are detected as the DEGs in the related-art technology but are not detected as the DEGs in the technology of the present disclosure. The genes C17orf64, CYP1A1, and SMG8 have almost no significant difference between the gene expression level distribution 30 of the high-expression group HG and the gene expression level distribution 30 of the low-expression group LG, as compared with the genes ESR1, GATA3, and TBC1D9 shown in Fig. 24. In addition, at present, there is no literature that reports the gene C17orf64, CYP1A1, and SMG8 as the DEGs. Therefore, the genes C17orf64, CYP1A1, and SMG8 can be said to be genes erroneously detected as the DEGs in the related-art technology. Therefore, the technology of the present disclosure can exclude the gene erroneously detected as the DEG in the related-art technology.

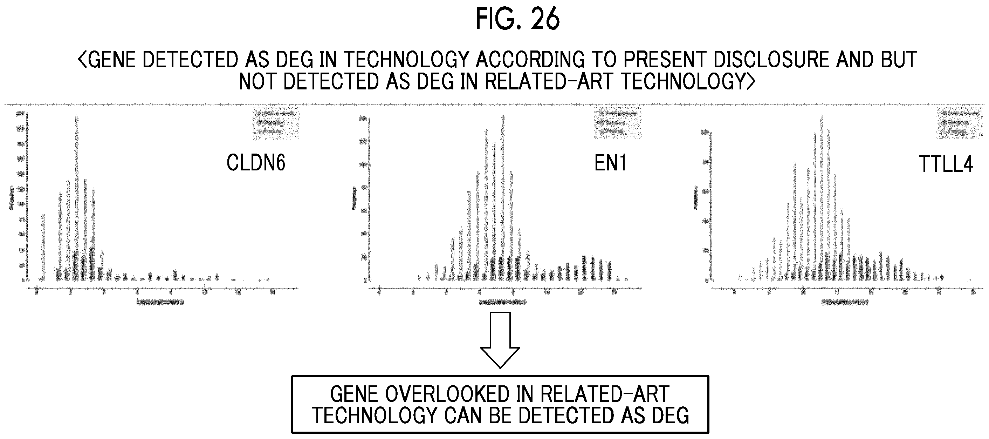

Fig. 26 shows the gene expression level distribution 30 of each of the genes CLDN6, EN1, and TTLL4 that are detected as the DEGs in the technology of the present disclosure but are not detected as the DEGs in the related-art technology, contrary to a case of Fig. 25. The genes CLDN6, EN1, and TTLL4 have the significant difference between the gene expression level distribution 30 of the high-expression group HG and the gene expression level distribution 30 of the low-expression group LG, as in the genes ESR1, GATA3, and TBC1D9 shown in Fig. 24. In addition, particularly in the gene expression level distribution 30 of the genes CLDN6 and EN1, the plurality of clusters appear.

Further, the genes CLDN6, EN1, and TTLL4 are reported as important genes in, for example, the following literatures B, C, and D.

Literature B <Y. Liu, et al., "DNA methylation of claudin-6 promotes breast cancer cell migration and invasion by recruiting mecp2 and deacetylating h3ac and h4ac." Journal of experimental & clinical cancer research, 35(1): 1-13, 2016.>

Literature C <G. Peluffo, et al., "En1 is a transcriptional dependency in triple-negative breast cancer associated with brain metastasis." Cancer research, 79 (16): 4173-4183, 2019.>

Literature D <J. Arnold, et al., "Tubulin tyrosine ligase like 4 (ttll4) overexpression in breast cancer cells is associated with brain metastasis and alters exosome biogenesis." Journal of Experimental & Clinical Cancer Research, 39 (1): 1-15, 2020.>

Therefore, it can be said that the genes CLDN6, EN1, and TTLL4 are genes that have been overlooked as the DEGs in the related-art technology. Therefore, the technology of the present disclosure can detect a gene overlooked as the DEG in the related-art technology, as the DEG.

From the above, it is confirmed that the technology of the present disclosure can detect the gene detected as the DEG in the related-art technology as the DEG in the same manner, can exclude a gene erroneously detected as the DEG in the related-art technology, and can detect the gene overlooked as the DEG in the related-art technology as the DEG. Therefore, it is proved that the technology of the present disclosure can detect an appropriate DEG even in a case in which the cell population in which the plurality of subtypes are mixed is the analysis target, as compared with the related-art technology.

The cell population is not limited to the cancer tissue-derived cell population 24 as an example. A population of human embryonic kidney 293 cells (HEK293 cells) which are human-derived cells for synthesizing a therapeutic protein or a virus used for gene therapy may be used. The cell characteristic of interest in a case of the HEK293 cell is the contribution to the synthesis of a therapeutic protein or a virus used for gene therapy. Further, the cells constituting the cell population are not limited to human cells. For example, a population of CHO cells may be used. The cell characteristic of interest in a case of the CHO cell is the contribution to antibody production.

The information processing device 10 may be the personal computer installed in the gene analysis facility as an example, or may be a server computer installed in a data center independent of the gene analysis facility.

In a case in which the information processing device 10 is configured by a server computer, the sample data group 11 is transmitted from the personal computer installed in each gene analysis facility to the server computer via a network such as the Internet. The server computer distributes various screens, such as the gene analysis screen 80, to the personal computer in a format of screen data for web distribution created by a markup language such as extensible markup language (XML). The personal computer reproduces a screen displayed on a web browser based on the screen data and displays the reproduced screen on the display. It should be noted that another data description language such as Javascript (registered trademark) object notation (JSON) may be used instead of the XML.

The hardware configuration of the computer constituting the information processing device 10 according to the technology of the present disclosure can be variously modified. For example, the information processing device 10 may be configured by a plurality of computers separated as hardware in order to improve processing capacity and reliability. For example, the functions of the acquisition unit 50, the distribution generation unit 51, and the display control unit 55 and the functions of the assignment unit 52, the search unit 53, and the detection unit 54 are distributed to two computers. In such a case, the information processing device 10 is configured by two computers.

As described above, the hardware configuration of the computer of the information processing device 10 can be changed as appropriate in accordance with required performance, such as processing capacity, safety, and reliability. Further, it goes without saying that, in addition to the hardware, an application program, such as the operation program 45, can be duplicated or distributed and stored in a plurality of storages for the purpose of securing the safety and the reliability.

In each of the above-described embodiments, for example, the following various processors can be used as a hardware structure of processing units that execute various types of processing, such as the acquisition unit 50, the distribution generation unit 51, the assignment units 52, 95, 100, and 105, the search units 53, 101, and 108, the detection unit 54, the display control unit 55, the function calculation unit 70, the score calculation unit 71, the determination unit 72, the evaluation unit 115, and the generation unit 120. As described above, the various processors include, in addition to the CPU 37, which is a general-purpose processor that executes software (operation program 45) to function as the various processing units, a programmable logic device (PLD), which is a processor of which a circuit configuration can be changed after the manufacturing, such as a field programmable gate array (FPGA), a dedicated electric circuit, which is a processor having a circuit configuration designed exclusively for executing specific processing, such as an application specific integrated circuit (ASIC), and the like.

One processing unit may be configured by one of these various processors, or may be configured by a combination of two or more processors of the same type or different types (for example, a combination of a plurality of FPGAs and/or a combination of a CPU and an FPGA). A plurality of processing units may be configured by one processor.

As an example in which the plurality of processing units are configured by one processor, first, as represented by a computer, such as a client and a server, there is a form in which one processor is configured by a combination of one or more CPUs and software, and the processor functions as the plurality of processing units. Second, as represented by a system on a chip (SoC) or the like, there is a form in which a processor, which implements the functions of the entire system including the plurality of processing units with a single integrated circuit (IC) chip, is used. As described above, as the hardware structure, the various processing units are configured by using one or more of the various processors described above.

Furthermore, as the hardware structure of the various processors, more specifically, an electric circuit (circuitry) in which circuit elements such as semiconductor elements are combined can be used.

The technology according to the following supplementary notes can be understood from the above description.

[Supplementary Note 1]

An information processing device that executes processing of detecting a differential expressed gene that exhibits a specific expression with respect to a cell characteristic of interest, based on gene expression level data of a cell population in which a plurality of subtypes are mixed, the information processing device comprising: a processor, in which the processor assigns a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene, and searches for a first probability distribution that fits the distributions of the gene expression levels of the two groups for each of the plurality of candidate genes, based on an assignment result of the clusters.

[Supplementary Note 2]

The information processing device according to supplementary note 1, in which the processor performs a determination of whether or not there is a statistically significant difference in the distribution of the gene expression levels between the two groups, based on a search result of the first probability distribution, for each of the plurality of candidate genes, and detects the candidate gene for which it is determined that there is the statistically significant difference in the distribution of the gene expression levels between the two groups, as the differential expressed gene.

[Supplementary Note 3]

The information processing device according to supplementary note 2, in which the processor calculates a representative index value for each of the two groups from the distribution of the gene expression levels and the searched first probability distribution, calculates a score representing a degree of a difference between the two groups from the representative index value, and performs the determination based on the score.

[Supplementary Note 4]

The information processing device according to any one of supplementary notes 1 to 3, in which the processor performs, at least once, processing of re-assigning the clusters by receiving a search result of the first probability distribution and re-searching for the first probability distribution based on a re-assignment result of the clusters.

[Supplementary Note 5]

The information processing device according to any one of supplementary notes 1 to 4, in which the processor changes the number of the clusters to be assigned, to search for the first probability distribution for each changed number, and determines a number in which the first probability distribution that best fits the distribution of the gene expression levels is searchable for from among the changed numbers, based on a search result of the first probability distribution.

[Supplementary Note 6]

The information processing device according to any one of supplementary notes 1 to 5, in which the processor assigns the clusters by using a second probability distribution.

[Supplementary Note 7]

The information processing device according to any one of supplementary notes 1 to 6, in which the first probability distribution is a negative binomial distribution.

[Supplementary Note 8]

The information processing device according to any one of supplementary notes 1 to 7, in which the processor generates simulated gene expression level data by simulation, based on the searched first probability distribution.

[Supplementary Note 9]

The information processing device according to supplementary note 8, in which the processor generates only the simulated gene expression level data of the differential expressed gene by the simulation.

The technology of the present disclosure can also be combined with various embodiments and/or various modification examples described above, as appropriate. In addition, it goes without saying that the present disclosure is not limited to each of the embodiments described above, various configurations can be adopted as long as the configuration does not deviate from the gist. Further, the technology of the present disclosure includes, in addition to the program, a storage medium that stores the program in a non-transitory manner.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In addition, in order to avoid complications and facilitate grasping the parts according to the technology of the present disclosure, in the above-described contents and the above-shown contents, the description of technical general knowledge and the like that do not particularly require description for enabling the implementation of the technology of the present disclosure are omitted.

In the present specification, "A and/or B" has the same meaning as "at least one of A or B". That is, "A and/or B" means that it may be only A, only B, or a combination of A and B. In addition, in the present specification, also in a case in which three or more matters are expressed in association by "and/or", the same concept as "A and/or B" is applied.

All of the documents, the patent applications, and the technical standards described in the present specification are incorporated herein by reference to the same extent as in a case in which each of the documents, patent applications, and technical standards is specifically and individually described by being incorporated by reference.

**Claims**

1. An information processing device that executes processing of detecting a differential expressed gene that exhibits a specific expression with respect to a cell characteristic of interest, based on gene expression level data of a cell population in which a plurality of subtypes are mixed, the information processing device comprising:

   a processor,
   wherein the processor

   assigns a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene, and searches for a first probability distribution that fits the distributions of the gene expression levels of the two groups for each of the plurality of candidate genes, based on an assignment result of the clusters.

2. The information processing device according to claim 1,
   wherein the processor

   performs a determination of whether or not there is a statistically significant difference in the distribution of the gene expression levels between the two groups, based on a search result of the first probability distribution, for each of the plurality of candidate genes, and
   detects the candidate gene for which it is determined that there is the statistically significant difference in the distribution of the gene expression levels between the two groups, as the differential expressed gene.

3. The information processing device according to claim 2,
   wherein the processor

   calculates a representative index value for each of the two groups from the distribution of the gene expression levels and the searched first probability distribution,
   calculates a score representing a degree of a difference between the two groups from the representative index value, and
   performs the determination based on the score.

4. The information processing device according to claim 1,
   wherein the processor performs, at least once, processing of re-assigning the clusters by receiving a search result of the first probability distribution and re-searching for the first probability distribution based on a re-assignment result of the clusters.

5. The information processing device according to claim 1,
   wherein the processor

   changes the number of the clusters to be assigned, to search for the first probability distribution for each changed number, and
   determines a number in which the first probability distribution that best fits the distribution of the gene expression levels is searchable for from among the changed numbers, based on a search result of the first probability distribution.

6. The information processing device according to claim 1,
   wherein the processor assigns the clusters by using a second probability distribution.

7. The information processing device according to claim 1,
   wherein the first probability distribution is a negative binomial distribution.

8. The information processing device according to claim 1,
   wherein the processor generates simulated gene expression level data by simulation, based on the searched first probability distribution.

9. The information processing device according to claim 8,

wherein the processor generates only the simulated gene expression level data of the differential expressed gene by the simulation.

10. An operation method of an information processing device that executes processing of detecting a differential expressed gene that exhibits a specific expression with respect to a cell characteristic of interest, based on gene expression level data of a cell population in which a plurality of subtypes are mixed, the operation method comprising:

assigning a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene; and searching for a first probability distribution that fits the distributions of the gene expression levels of the two groups for each of the plurality of candidate genes, based on an assignment result of the clusters.

11. An operation program of an information processing device that executes processing of detecting a differential expressed gene that exhibits a specific expression with respect to a cell characteristic of interest, based on gene expression level data of a cell population in which a plurality of subtypes are mixed, the operation program causing a computer to execute a process comprising:

assigning a cluster to which each sample of two groups obtained by dividing the cell population in accordance with the cell characteristic of interest is estimated to belong in a distribution of gene expression levels, to each sample, for each of a plurality of candidate genes that are candidates for the differential expressed gene; and searching for a first probability distribution that fits the distributions of the gene expression levels of the two groups for each of the plurality of candidate genes, based on an assignment result of the clusters.

# FIG. 1

ER STATE INFORMATION 16
SAMPLE ID: S0001
HIGH EXPRESSION/LOW EXPRESSION

GENE EXPRESSION LEVEL DATA 17
SAMPLE ID: S0001

| GENE | GENE EXPRESSION LEVEL |
|------|------------------------|
| ⋮ | ⋮ |
| ESR1 | 14.7 |
| GATA3 | 12.2 |
| CYP1A1 | 2.6 |
| TTLL4 | 4.5 |
| ⋮ | ⋮ |

GENE EXPRESSION ANALYSIS

ER STATE INFORMATION 16

GENE EXPRESSION LEVEL DATA 17

EP 4 610 986 A1

FIG. 2

GENE EXPRESSION LEVEL DISTRIBUTION

# FIG. 3

39

DISPLAY ~12

INPUT DEVICE ~13

10

CPU ~37

MEMORY ~36

STORAGE ~35

COMMUNICATION UNIT ~38

# FIG. 4

# FIG. 5

13 — INPUT DEVICE

65 — NUMBER-OF-CLUSTERS SETTING INFORMATION

K = 2

52 — ASSIGNMENT UNIT

60 — ASSIGNMENT RESULT

| SAMPLE ID | GROUP | CLUSTER |
|-----------|-------|---------|
| S0001 | HIGH-EXPRESSION GROUP | 1 |
| S0002 | LOW-EXPRESSION GROUP | 1 |
| S0003 | LOW-EXPRESSION GROUP | 2 |
| ⋮ | | |

53 — SEARCH UNIT

## FIG. 6

$$Y_{i\,ESR1}^{(LG)} = Y_{i\,ESR1\,C1}^{(LG)} + Y_{i\,ESR1\,C2}^{(LG)} \; TO \; P_{NB} \left( p_{ESR1\,C1}^{(LG)} , r_{ESR1\,C1}^{(LG)} \right) + P_{NB} \left( p_{ESR1\,C2}^{(LG)} , r_{ESR1\,C2}^{(LG)} \right)$$

NUMBER OF SAMPLES

ESR1

30

EXPRESSION LEVEL

$$Y_{i\,ESR1}^{(HG)} = Y_{i\,ESR1\,C1}^{(HG)} + Y_{i\,ESR1\,C2}^{(HG)}$$

$$TO \; P_{NB} \left( p_{ESR1\,C1}^{(HG)} , r_{ESR1\,C1}^{(HG)} \right) + P_{NB} \left( p_{ESR1\,C2}^{(HG)} , r_{ESR1\,C2}^{(HG)} \right)$$

$Y_{i\,ESR1}^{(HG)}$: COUNT DATA OF GENE ESR1 OF SAMPLE i OF HIGH-EXPRESSION GROUP

$Y_{i\,ESR1\,C1}^{(HG)}$: COUNT DATA OF SAMPLE i ESTIMATED TO BELONG TO CLUSTER 1 IN HIGH-EXPRESSION GROUP

$Y_{i\,ESR1\,C2}^{(HG)}$: COUNT DATA OF SAMPLE i ESTIMATED TO BELONG TO CLUSTER 2 IN HIGH-EXPRESSION GROUP

$Y_{i\,ESR1}^{(LG)}$: COUNT DATA OF GENE ESR1 OF SAMPLE I OF LOW-EXPRESSION GROUP

$Y_{i\,ESR1\,C1}^{(LG)}$: COUNT DATA OF SAMPLE i ESTIMATED TO BELONG TO CLUSTER 1 IN LOW-EXPRESSION GROUP

$Y_{i\,ESR1\,C2}^{(LG)}$: COUNT DATA OF SAMPLE i ESTIMATED TO BELONG TO CLUSTER 2 IN LOW-EXPRESSION GROUP

$P_{NB}$: NEGATIVE BINOMIAL DISTRIBUTION

p,r: PARAMETERS OF NEGATIVE BINOMIAL DISTRIBUTION

EP 4 610 986 A1

# FIG. 7

SEARCH RESULT — 61

| CANDIDATE GENE | GROUP | SEARCHED BINOMIAL DISTRIBUTION |
|---|---|---|
| | | $\vdots$ |
| ESR1 | HIGH-EXPRESSION GROUP | $P_{NB}\left(p^{(HG)}_{ESR1\ C1}, r^{(HG)}_{ESR1\ C1}\right), P_{NB}\left(p^{(HG)}_{ESR1\ C2}, r^{(HG)}_{ESR1\ C2}\right)$ |
| | LOW-EXPRESSION GROUP | $P_{NB}\left(p^{(LG)}_{ESR1\ C1}, r^{(LG)}_{ESR1\ C1}\right), P_{NB}\left(p^{(LG)}_{ESR1\ C2}, r^{(LG)}_{ESR1\ C2}\right)$ |
| GATA3 | HIGH-EXPRESSION GROUP | $P_{NB}\left(p^{(HG)}_{GATA3\ C1}, r^{(HG)}_{GATA3\ C1}\right), P_{NB}\left(p^{(HG)}_{GATA3\ C2}, r^{(HG)}_{CATA3\ C2}\right)$ |
| | LOW-EXPRESSION GROUP | $P_{NB}\left(p^{(LG)}_{GATA3\ C1}, r^{(LG)}_{GATA3\ C1}\right), P_{NB}\left(p^{(LG)}_{GATA3\ C2}, r^{(LG)}_{GATA3\ C2}\right)$ |
| CYP1A1 | HIGH-EXPRESSION GROUP | $P_{NB}\left(p^{(HG)}_{CYP1A1\ C1}, r^{(HG)}_{CYP1A1\ C1}\right), P_{NB}\left(p^{(HG)}_{CYP1A1\ C2}, r^{(HG)}_{CYP1A1\ C2}\right)$ |
| | LOW-EXPRESSION GROUP | $P_{NB}\left(p^{(LG)}_{CYP1A1\ C1}, r^{(LG)}_{CYP1A1\ C1}\right), P_{NB}\left(p^{(LG)}_{CYP1A1\ C2}, r^{(LG)}_{CYP1A1\ C2}\right)$ |
| TTLL4 | HIGH-EXPRESSION GROUP | $P_{NB}\left(p^{(HG)}_{TTLL4\ C1}, r^{(HG)}_{TTLL4\ C1}\right), P_{NB}\left(p^{(HG)}_{TTLL4\ C2}, r^{(HG)}_{TTLL4\ C2}\right)$ |
| | LOW-EXPRESSION GROUP | $P_{NB}\left(p^{(LG)}_{TTLL4\ C1}, r^{(LG)}_{TTLL4\ C1}\right), P_{NB}\left(p^{(LG)}_{TTLL4\ C2}, r^{(LG)}_{TTLL4\ C2}\right)$ |
| | | $\vdots$ |

EP 4 610 986 A1

## FIG. 8

START

ST10 — DETERMINE WHETHER OR NOT THERE IS STATISTICALLY SIGNIFICANT DIFFERENCE BETWEEN GENE EXPRESSION LEVEL DISTRIBUTION OF HIGH-EXPRESSION GROUP AND GENE EXPRESSION LEVEL DISTRIBUTION OF LOW-EXPRESSION GROUP BASED ON SEARCH RESULT

ST20 — IS IT DETERMINED THAT THERE IS STATISTICALLY SIGNIFICANT DIFFERENCE?   NO

ST30 — YES
DETECT CANDIDATE GENE AS DEG

ST40 — NO   IS DETERMINATION FOR ALL CANDIDATE GENE COMPLETED?

YES

END

EP 4 610 986 A1

# FIG. 9

31 GENE EXPRESSION LEVEL DISTRIBUTION GROUP

61 SEARCH RESULT

DETECTION UNIT

FUNCTION CALCULATION UNIT — 70

54

FUNCTION CALCULATION RESULT — 75

SCORE CALCULATION UNIT — 71

SCORE CALCULATION RESULT — 76

DETERMINATION UNIT — 72

DETECTION RESULT — 62

## FIG. 10

75

| CANDIDATE GENE | GROUP | CALCULATED LOGARITHMIC LIKELIHOOD FUNCTION |
|---|---|---|
| | | $\vdots$ |
| ESR1 | HIGH-EXPRESSION GROUP | $I^{(HG)}_{ESR1}$ |
| | LOW-EXPRESSION GROUP | $I^{(LG)}_{ESR1}$ |
| | BOTH GROUPS (WHOLE) | $I_{ESR1}$ |
| GATA3 | HIGH-EXPRESSION GROUP | $I^{(HG)}_{GATA3}$ |
| | LOW-EXPRESSION GROUP | $I^{(LG)}_{GATA3}$ |
| | BOTH GROUPS (WHOLE) | $I_{GATA3}$ |
| | | $\vdots$ |

FUNCTION CALCULATION RESULT

## FIG. 11

76

| CANDIDATE GENE | CALCULATED SCORE |
|---|---|
| | $\vdots$ |
| ESR1 | $\Lambda_{ESR1} (=-2(I_{ESR1} - I^{(HG)}_{ESR1} - I^{(LG)}_{ESR1}))$ |
| GATA3 | $\Lambda_{GATA3} (=-2(I_{GATA3} - I^{(HG)}_{GATA3} - I^{(LG)}_{GATA3}))$ |
| | $\vdots$ |

SCORE CALCULATION RESULT

## FIG. 12

ST10

```
                    ┌─────────────┐
                    │    START    │
                    └──────┬──────┘
                           │
ST101┐                     ▼
┌──────────────────────────────────────────┐
│ PERFORM LIKELIHOOD RATIO TEST IN          │
│ ACCORDANCE WITH NULL HYPOTHESIS THAT      │
│ THERE IS NO STATISTICALLY SIGNIFICANT     │
│ DIFFERENCE BETWEEN GENE EXPRESSION        │
│ LEVEL DISTRIBUTION OF HIGH-EXPRESSION     │
│ GROUP AND GENE EXPRESSION LEVEL           │
│ DISTRIBUTION OF LOW-EXPRESSION GROUP,     │
│ AND CALCULATE p-VALUE                     │
└──────────────────────────────────────────┘
ST102┐                     │
┌──────────────────────────────────────────┐
│           CORRECT p-VALUE                 │
└──────────────────────────────────────────┘
ST103┐                     │
```

IS CORRECTED p-VALUE LESS THAN PRESET SIGNIFICANCE LEVEL?  NO

ST104┐  YES

REJECT NULL HYPOTHESIS AND DETERMINE THAT THERE IS STATISTICALLY SIGNIFICANT DIFFERENCE

ST105┐

ADOPT NULL HYPOTHESIS AND DETERMINE THAT THERE IS NO STATISTICALLY SIGNIFICANT DIFFERENCE

END

EP 4 610 986 A1

# FIG. 13

GENE ANALYSIS — 80, 81

CELL POPULATION: CANCER TISSUE-DERIVED CELLS    NUMBER OF SAMPLES: 1222
SAMPLED FROM BRCA PATIENT
GROUPING: ER HIGH EXPRESSION, LOW EXPRESSION

DEG DETECTION — 84

82 — DEG DETECTION RESULT — 83, 86

ESR1 — 30, 85

GATA3 — 30, 85

TBC1D9 — 30, 85

CLDN6 — 30, 85

# FIG. 14

GENE EXPRESSION LEVELS OF ALL GENES

GENE EXPRESSION LEVEL OF DEG

ER STATE
PREDICTION MODEL ~90

ER STATE
PREDICTION RESULT
HIGH
EXPRESSION/LOW
EXPRESSION ~91

# FIG. 15

START

ST1000 → ACQUIRE SAMPLE DATA GROUP

ST1100 → IS DEG DETECTION INSTRUCTION ISSUED? — NO

ST1200 → GENERATE GENE EXPRESSION LEVEL DISTRIBUTION OF EACH CANDIDATE GENE

YES

ST1300 → ASSIGN CLUSTER TO EACH SAMPLE

ST1400 → SEARCH FOR NEGATIVE BINOMIAL DISTRIBUTION THAT FITS GENE EXPRESSION LEVEL DISTRIBUTION

ST1500 → DETECT DEG

ST1600 → DISPLAY DETECTION RESULT OF DEG

END

FIG. 16

95 — ASSIGNMENT UNIT

ASSIGN CLUSTER TO EACH SAMPLE USING one-hot VECTOR zi SAMPLED FROM CATEGORICAL DISTRIBUTION $P_{Cat}(\pi^{(j)})$ IN WHICH K-DIMENSIONAL VECTOR $\pi^{(j)}$ FOLLOWS DIRICHLET DISTRIBUTION $P_{Dir}(\alpha)$

60 —

**ASSIGNMENT RESULT**

| SAMPLE ID | GROUP | CLUSTER |
|-----------|-------|---------|
| S0001 | HIGH-EXPRESSION GROUP | 1 |
| S0002 | LOW-EXPRESSION GROUP | 1 |
| S0003 | LOW-EXPRESSION GROUP | 2 |
| ⋮ | | |

53 — SEARCH UNIT

EP 4 610 986 A1

## FIG. 17

100 — ASSIGNMENT UNIT

60 — ASSIGNMENT RESULT

101 — SEARCH UNIT

61 — SEARCH RESULT

## FIG. 18

ST1200
┌─────────────────────────────────┐
│ GENERATE GENE EXPRESSION LEVEL   │
│ DISTRIBUTION OF EACH CANDIDATE GENE │
└─────────────────────────────────┘

ST1300
┌─────────────────────────────────┐
│     ASSIGN CLUSTER TO EACH SAMPLE     │
└─────────────────────────────────┘

ST1400
┌─────────────────────────────────┐
│      SEARCH FOR NEGATIVE BINOMIAL      │
│ DISTRIBUTION THAT FITS GENE EXPRESSION │
│        LEVEL DISTRIBUTION        │
└─────────────────────────────────┘

ST1420
┌──────────────────────────────────────┐
│ CHANGE ASSIGNMENT METHOD OF CLUSTERS  │
│        BASED ON SEARCH RESULT        │
└──────────────────────────────────────┘

ST1410
IS END CONDITION SATISFIED? — NO

YES

ST1500
┌─────────────────────────────────┐
│           DETECT DEG            │
└─────────────────────────────────┘

EP 4 610 986 A1

## FIG. 19

| NUMBER-OF-CLUSTERS SETTING INFORMATION | K = 2 | (106A) |
| NUMBER-OF-CLUSTERS SETTING INFORMATION | K = 3 | (106B) |
| NUMBER-OF-CLUSTERS SETTING INFORMATION | K = 4 | (106C) |

105 — ASSIGNMENT UNIT

### 107A — ASSIGNMENT RESULT (K = 2)

| SAMPLE ID | GROUP | CLUSTER |
|---|---|---|
| S0001 | HIGH-EXPRESSION GROUP | 1 |
| S0002 | LOW-EXPRESSION GROUP | 1 |
| S0003 | LOW-EXPRESSION GROUP | 2 |
| ⋮ | | |

### 107B — ASSIGNMENT RESULT (K = 3)

| SAMPLE ID | GROUP | CLUSTER |
|---|---|---|
| S0001 | HIGH-EXPRESSION GROUP | 1 |
| S0002 | LOW-EXPRESSION GROUP | 3 |
| S0003 | LOW-EXPRESSION GROUP | 2 |
| ⋮ | | |

### 107C — ASSIGNMENT RESULT (K = 4)

| SAMPLE ID | GROUP | CLUSTER |
|---|---|---|
| S0001 | HIGH-EXPRESSION GROUP | 1 |
| S0002 | LOW-EXPRESSION GROUP | 4 |
| S0003 | LOW-EXPRESSION GROUP | 3 |
| ⋮ | | |

108 — SEARCH UNIT

SEARCH RESULT (K = 2) — 109A   SEARCH RESULT (K = 3) — 109B   SEARCH RESULT (K = 4) — 109C

# FIG. 20

SEARCH RESULT
(K = 2)
109A

SEARCH RESULT
(K = 3)
109B

SEARCH RESULT
(K = 4)
109C

115 — EVALUATION UNIT

31 — GENE EXPRESSION LEVEL DISTRIBUTION GROUP

116 — EVALUATION RESULT

| NUMBER-OF-CLUSTERS SETTING VALUE K | 2 | 3 | 4 |
|---|---|---|---|
| EVALUATION OF SEARCH RESULT | IMPOSSIBLE | GOOD | POSSIBLE |

105 — ASSIGNMENT UNIT

DETERMINE K = 3

## FIG. 21

61 — SEARCH RESULT

120 — GENERATION UNIT ⎯⎯ GENERATE SIMULATED GENE EXPRESSION LEVEL DATA FROM SEARCH RESULT BY SIMULATION

SIMULATED GENE EXPRESSION LEVEL DATA

121

SIMULATED GENE EXPRESSION LEVEL DATA

121

SIMULATED GENE EXPRESSION LEVEL DATA ...

121

EP 4 610 986 A1

FIG. 22

EP 4 610 986 A1

## FIG. 23

EP 4 610 986 A1

| GROUP / SUBTYPE | HIGH EXPRESSION | LOW EXPRESSION | INTERMEDIATE (CANNOT BE CLASSIFIED) | NaN (NO LABEL) | Total |
|---|---|---|---|---|---|
| Basal-like | 21 | 142 | 0 | 7 | 170 |
| Her2-enriched | 26 | 45 | 2 | 4 | 77 |
| Luminal A | 463 | 11 | 0 | 19 | 493 |
| Luminal B | 177 | 3 | 0 | 12 | 192 |
| Normal-like | 20 | 12 | 0 | 3 | 35 |
| NaN (NO LABEL) | 190 | 48 | 1 | 16 | 255 |
| Total | 897 | 261 | 3 | 61 | 1222 |

125

## FIG. 24

### ⟨GENE DETECTED AS DEG IN BOTH RELATED-ART TECHNOLOGY
### AND TECHNOLOGY ACCORDING TO PRESENT DISCLOSURE⟩

ESR1    GATA3    TBC1D9

DEG THAT IS CONSISTENT WITH PRIOR RESEARCH
CAN BE DETECTED IN BOTH RELATED-ART
TECHNOLOGY AND TECHNOLOGY ACCORDING TO
PRESENT DISCLOSURE

# FIG. 25

<GENE DETECTED AS DEG IN RELATED-ART TECHNOLOGY AND BUT NOT DETECTED
AS DEG IN TECHNOLOGY ACCORDING TO PRESENT DISCLOSURE>

C17orf64

CYP1A1

SMG8

GENE ERRONEOUSLY DETECTED AS DEG IN
RELATED-ART TECHNOLOGY CAN BE EXCLUDED

EP 4 610 986 A1

## FIG. 26

<GENE DETECTED AS DEG IN TECHNOLOGY ACCORDING TO PRESENT DISCLOSURE AND BUT
NOT DETECTED AS DEG IN RELATED-ART TECHNOLOGY>

GENE OVERLOOKED IN RELATED-ART
TECHNOLOGY CAN BE DETECTED AS DEG

## INTERNATIONAL SEARCH REPORT

| International application No. |
| --- |
| **PCT/JP2023/043019** |

**A. CLASSIFICATION OF SUBJECT MATTER**

*G16B 25/00*(2019.01)i; *C12M 1/00*(2006.01)i; *C12M 1/34*(2006.01)i; *C12N 15/09*(2006.01)i
FI: G16B25/00; C12M1/00 A; C12M1/34 D; C12N15/09 Z

According to International Patent Classification (IPC) or to both national classification and IPC

**B. FIELDS SEARCHED**

Minimum documentation searched (classification system followed by classification symbols)

G16B25/00; C12M1/00; C12M1/34; C12N15/09

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2005-157500 A (INTERNATL BUSINESS MACH CORP <IBM>) 16 June 2005 (2005-06-16) <br> entire text, all drawings | 1-11 |
| A | JP 2018-139043 A (UNIV RYUKOKU) 06 September 2018 (2018-09-06) <br> entire text, all drawings | 1-11 |
| A | JP 2020-190935 A (FUJITSU LTD) 26 November 2020 (2020-11-26) <br> entire text, all drawings | 1-11 |
| A | CN 112837754 A (ANALYTICAL BIOSCIENCES LIMITED) 25 May 2021 (2021-05-25) <br> entire text, all drawings | 1-11 |

☐ Further documents are listed in the continuation of Box C.    ☑ See patent family annex.

| | |
| --- | --- |
| *   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
| --- | --- |
| **18 January 2024** | **30 January 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
Information on patent family members

International application No.

**PCT/JP2023/043019**

| Patent document cited in search report | | | Publication date (day/month/year) | Patent family member(s) | | | Publication date (day/month/year) |
|---|---|---|---|---|---|---|---|
| JP | 2005-157500 | A | 16 June 2005 | US | 2005/0112670 | A1 | |
| | | | | entire text, all drawings | | | |
| JP | 2018-139043 | A | 06 September 2018 | (Family: none) | | | |
| JP | 2020-190935 | A | 26 November 2020 | US | 2020/0373012 | A1 | |
| | | | | entire text, all drawings | | | |
| CN | 112837754 | A | 25 May 2021 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Y. LIU et al.** DNA methylation of claudin-6 promotes breast cancer cell migration and invasion by recruiting mecp2 and deacetylating h3ac and h4ac.. *Journal of experimental & clinical cancer research*, 2016, vol. 35 (1), 1-13
- **G. PELUFFO et al.** En1 is a transcriptional dependency in triple-negative breast cancer associated with brain metastasis.. *Cancer research*, 2019, vol. 79 (16), 4173-4183
- **J. ARNOLD et al.** Tubulin tyrosine ligase like 4 (ttll4) overexpression in breast cancer cells is associated with brain metastasis and alters exosome biogenesis.. *Journal of Experimental & Clinical Cancer Research*, 2020, vol. 39 (1), 1-15